# EUROPÄISCHE PATENTSCHRIFT

(11) **EP 2 679 269 B1**
(45) Veröffentlichungstag und Bekanntmachung des Hinweises auf die Patenterteilung: **14.07.2021**
(21) Anmeldenummer: 13176615.6
(22) Anmeldetag: 12.06.2006
(51) Int. Cl.: A61M 25/06, A61M 5/158, A61M 5/32

(54) **Insertionskopf mit Griff**
Insertion head with handle
Tête d'insertion avec poignée

(30) Priorität: 15.09.2005 EP 05020156
(43) Veröffentlichungstag der Anmeldung: 01.01.2014
(62) Teilanmeldung aus: 06762013.8
(73) Patentinhaber: F. Hoffmann-La Roche AG, 4070 Basel (CH); Roche Diabetes Care GmbH, 68305 Mannheim (DE)
(72) Erfinder: Scheurer, Simon, 3006 Bern (CH); Thalmann, Christian, 6365 Kehrsiten (CH)
(74) Vertreter: Rentsch Partner AG

(56) Entgegenhaltungen:
- WO-A-02/02165
- WO-A1-97/21457
- DE-U1- 20 320 207
- DE-U1-202004 017 861
- FR-A- 2 752 164
- None

## Beschreibung

Die Erfindung betrifft einen Insertionskopf für medizinische oder pharmazeutische Anwendungen, der auf einem organischen Gewebe, vorzugsweise der menschlichen Haut, platzierbar ist und eine Einführeinrichtung aufweist, die in das Gewebe eindringt, wenn der Insertionskopf auf dem Gewebe platziert wird oder gegebenenfalls auch erst nachdem der Insertionskopf auf dem Gewebe platziert wurde. Der Insertionskopf kann insbesondere Bestandteil eines Infusionssets für die Verabreichung eines Medikaments sein.

Aus der DE 198 21 723 C1 ist ein Insertionskopf bekannt, der eine Basis, eine flexible Kanüle und eine Einstechnadel umfasst. Die Kanüle ragt von einer Unterseite der Basis ab. Die Einstechnadel stabilisiert die Kanüle, während die Kanüle in das Gewebe eines Patienten eingeführt wird. Für die Stabilisierung durchragt die Einstechnadel die Kanüle, und die Kanüle umschmiegt die Einstechnadel. Als Schutz vor Stichverletzungen ist an der Basis lösbar ein Nadelschutz befestigt. Die von der Unterseite des Insertionskopfs abragende Einstechnadel mit der umgebenden Kanüle und insbesondere auch der Nadelschutz vergrößern das Volumen des Insertionskopfs und dadurch auch dessen Verpackung beträchtlich. Ferner ist das Entfernen des Nadelschutzes umständlich.

Die deutsche Patentanmeldung Nr. 10 2004 039 408.3 hat einen Insertionskopf zum Gegenstand, der eine Basis mit einer auf organischem Gewebe platzierbaren Unterseite und platzsparend eine in das Gewebe einführbare, von der Basis beweglich gelagerte Einführeinrichtung umfasst. Für die Lagerung, den Transport und die Handhabung bis zur Einführung in das Gewebe nimmt die Einführeinrichtung eine Schutzposition ein. Für das Einführen ist sie aus der Schutzposition in eine Einführposition bewegbar. Als bevorzugte Art der Bewegung wird eine Schwenkbeweglichkeit offenbart. Um die Bewegung der Einführeinrichtung bewirken zu können, ist für den Benutzer ein Griff vorgesehen, der gemeinsam mit der Einführeinrichtung schwenkbar ist. Der Insertionskopf ist vorteilhaft kompakt und erfordert auch nicht das Entfernen eines Nadelschutzes. Um die Einführeinrichtung in die Einführposition zu bewegen, muss der Benutzer jedoch mit der einen Hand den Insertions-kopf greifen und mit der anderen Hand den Griff mit der Einführeinrichtung schwenken.

DE 20200401 7861 U1 zeigt eine Ausziehvorrichtung zum geschützten Herausziehen einer flexiblen Punktionsnadel aus einer flexiblen Infusionskanüle. Ein Insertionskopf wird auf der Körperoberfläche angebracht, und die aus der Unterseite des Insertionskopfs hervorstehende und durch die Punktionsnadel versteifte Infusionskanüle ins Gewebe eingeführt. Die Punktionsnadel ist durch ein Septum einer Verbindungsvorrichtung geführt, und ist an ihrem distalen Ende mit einer Spule mit einem Zahnrad verbunden. Spule und Zahnrad ist auf einem am Insertionskopf linear verschiebbar gelagerten Rückzugshebel drehbar gelagert. Das Zahnrad greift in eine in Bezug auf den Insertionskopf feststehende Zahnschiene ein, wodurch durch das manuelle Zurückziehen des Rückzughebels in Bezug auf den Insertionskopf zum einen die Punktionsnadel aus der Kanüle gezogen wird, und zum anderen die Punktionskanüle zu einem gewissen Teil auf der Spule aufgewickelt wird. Rückzugshebel und Zahnschiene und die darin geschützte Punktionsnadel werden anschliessend vom Insertionskopf gelöst und entsorgt.

WO 97/21457 A1 zeigt in den Figuren 13 bis 15 eine Infusionsvorrichtung miteiner Grundplatte, welche auf der Hautoberfläche angebracht wird. Eine mit einem beweglichen Kolben und einem Septum versehene Ampulle mit dem flüssigen Medikament ist an einem dem Septum entgegen gesetzten Ende auf einem schwenkbar auf der Grundplatte befestigten Montageelement linear verschiebbar gelagert. An dem mit dem Septum versehenen Ende der Ampulle ist ein Nadelträger mit einer Injektions-Hohlnadel mit zwei zugespitzten Enden entlang der Längsachse der Ampulle linear verschiebbar gelagert. Auf der Grundplatte ist weiter ein Hebel schwenkbar gelagert, wobei der Nadelträger drehbeweglich mit dem Hebel verbunden ist. Durch Schwenken des Hebels von einer ersten Position im Wesentlichen senkrecht zur Grundplatte hin zu einer zweiten Position im Wesentlichen parallel zur Grundplatte wird das eine Ende der Injektions-Hohlnadel ins Gewebe eingeführt. Gleichzeitig wird die Hohlnadel entlang der Ampullenachse verschoben, so dass das andere Ende der Hohlnadel das Septum durchsticht und so die Injektionsnadel mit dem Ampulleninneren verbindet. Gleichzeitig wird die Ampulle auf das Montageelement geschoben, wodurch eine Gastreibladung aus Zitronensäure und Natriumbikarbonat aktiviert wird, welche den Kolben nach vorne treibt und so das flüssige Medikament durch die Injektionsnadel fördert.

WO 02/02165 A2 zeigt eine Inserter-Vorrichtung, die mit einer Grundplatte auf der Körperoberfläche eines Patienten angebracht ist. Durch Drücken eines Insertionskopfs mit einem Nadelträger mit einer festen Injektionsnadel gegen die Grundplatte wird die Nadel durch eine Öffnung in der Grundplatte ins Gewebe des Patienten eingeführt. Gleichzeitig wird eine Rückstellfeder vorgespannt. Wird die Grundplatte von der Körperoberfläche entfernt, so wird die Rückstellfeder entriegelt, welche anschliessend den Nadelträger zurückschiebt, wodurch die Injektionsnadel aus der Gewebe gezogen und hinter die Unterseite der Bodenplatte zurückgezogen wird.

DE 20320207 U1 zeigt einen Inserter zur Platzierung eines Insertionskopfes, mit einer Vortriebseinrichtung zum Bewegen des in einer Aufnahme gehaltenen Insertionskopfes in eine Vortriebsrichtung. Die Vortriebseinrichtung ist so ausgebildet, dass die Bewegung der Aufnahme mit dem Insertionskopf in Bezug auf das Insertergehäuse gleitfrei erfolgt. Die Vortriebsvorrichtung umfasst zwei Paare von gelenkig miteinander verbundenen Schenkeln. Die beiden Schenkelpaare sind symmetrisch zur Vortriebsrichtung angeordnet. Jedes der beiden Schenkelpaare ist in einem zum Insertergehäuse ortsfesten Drehgelenk aufgehängt. Die beiden Schenkel des jeweiligen Schenkelpaares sind in einem freien Drehgelenk miteinander drehbeweglich verbunden. Der von dem ortsfesten Gelenk abgewandte Schenkel jedes Schenkelpaares ist jeweils in einem weiteren Drehgelenk mit der Aufnahme für den Insertionskopf verbunden.

FR 2752164 A1 zeigt einen Insertionskopf zum temporären Verbinden eines implantierten Portkatheters mit einer externen Zuflussleitung. Der Insertionskopf umfasst zwei Griffteile, die gegen eine rückstellende Federkraft ineinandergeschoben werden können. Einefeste Injektionsnadel ist an einer Achse befestigt, die im einen Griffteil drehbar gelagert ist. Die Achse weist Kulissensteine auf, die mit Kulissen im anderen Griffelement wechselwirken, so dass ein lineares Ineinanderschieben der Griffelemente durch Muskelkraft bzw. Auseinanderdrücken durch Federkraft eine Drehung der Achse und eine Schwenkbewegung der Injektionsnadel zwischen einer Position innerhalb des Griffgehäuses und einer Position senkrecht zur Unterseite des Griffgehäuses bewirkt.

Es ist eine Aufgabe der der Erfindung, einen Insertionskopf zu schaffen, der einen integrierten Schutz für eine Einführeinrichtung aufweist, beispielsweise dem Insertionskopf aus DE1 02004039408 A1 vergleichbar, aber einfacher handhabbar ist.

Ein Insertionskopf, wie die Erfindung ihn betrifft ist in Anspruch 1 angegeben. Der Insertionskopf umfasst eine Basis mit einer auf organischem Gewebe platzierbaren Unterseite und eine in das Gewebe einführbare, von der Basis beweglich gelagerte Einführeinrichtung, die relativ zu der Basis aus einer Schutzposition in eine Einführposition bewegbar ist. In der Schutzposition steht zumindest ein freies Ende der Einführeinrichtung hinter der Unterseite der Basis zurück. Vorzugsweise steht die Einführeinrichtung in der Schutzposition über ihre gesamte Länge hinter der Unterseite der Basis zurück und wird vollständig abgeschirmt, vorzugsweise auch blickdicht verdeckt. In bevorzugten Ausführungen weist die Einführeinrichtung in der Schutzposition zumindest im Wesentlichen parallel zu der Unterseite bzw. der Auflagefläche der Basis. Dies begünstigt eine flache Bauweise der Basis, wobei deren Höhe rechtwinklig zur Unterseite gemessen wird. In der Einführposition steht das freie Ende über die Unterseite vor und kann in das Gewebe eingeführt werden. Die Einführeinrichtung kann eine biegesteife Kanüle oder Nadel sein. Bevorzugt ist die Einführeinrichtung zumindest in dem Gewebe flexibel. Insbesondere kann die Einführeinrichtung eine Biegesteifigkeit aufweisen, die sich im eingeführten Zustand aufgrund einer zwischen dem Material der Einführeinrichtung und dem umgebenden Gewebe stattfindenden Wechselwirkung verringert. Die Einführeinrichtung kann alternativ auch in herkömmlicher Weise flexibel gebildet sein, beispielsweise als flexible Kanüle, und von einer biegesteifen Einstecheinrichtung während der Einführung in das Gewebe stabilisiert werden. Die Einführeinrichtung ist vorzugsweise in eine Einführrichtung langgestreckt und vorzugsweise schlank.

Die Einführeinrichtung ragt in der Einführungsposition vorzugsweise von der Unterseite des Gehäuses vor. Grundsätzlich kann sie jedoch stattdessen auch von einer Seite des Gehäuses vorragen, solange sie für ein Eindringen in das Gewebe über die Unterseite ausreichend weit vorragt. Die Einführeinrichtung ragt vorzugsweise mit einer an subkutane Applikationen angepassten Länge über die Unterseite des Gehäuses vor, vorzugsweise unmittelbar von der Unterseite ab oder aus der Unterseite hervor. Für Applikationen innerhalb der Haut oder in intramuskulärem Gewebe ist die Einführeinrichtung entsprechend kürzer oder länger. Als Einführeinrichtung wird derjenige Längenabschnitt verstanden, der in der Applikation in das Gewebe ragt.

In der Lage, welche die Einführeinrichtung in dem Moment einnimmt, wenn ihr freies Ende über die Unterseite vorschwenkt, schließt die Längsachse der Einführeinrichtung mit der Unterseite der Basis einen spitzen Winkel von vorzugsweise weniger als 50° ein. Vorzugsweise ist der Winkel kleiner als 30°, so dass die Längsachse bzw. die Einführeinrichtung im Moment des Ausschwenkens zumindest im Wesentlichen parallel zu der Unterseite bzw. der Auflagefläche der Basis weist.

Die Längsachse der Einführeinrichtung, die um die Rotationsachse geschwenkt wird, schneidet die Rotationsachse vorzugsweise. Falls die Längsachse der Einführeinrichtung die Rotationsachse nicht schneidet, sondern in einem Abstand kreuzt, ist der Abstand vorzugsweise deutlich kleiner als die Länge der Einführeinrichtung. Vorzugsweise ist der Abstand höchstens halb so groß wie die Eindringtiefe bzw. Länge der Einführeinrichtung. Der Schwenkwinkel der Einführeinrichtung beträgt in bevorzugten Ausführungen 90° ± 10°. In ebenfalls vorteilhaften Ausführungen kann der Schwenkwinkel jedoch auch kleiner sein, insbesondere falls die Einführeinrichtung in der Einführposition nicht rechtwinklig zur Unterseite der Basis weist, sondern in einem spitzen Winkel, der allerdings wenigstens 30° betragen sollte. Entsprechend beträgt der Schwenkwinkel in derartigen Ausführungen vorzugsweise wenigstens etwa 30° oder jeden Zwischenwert zwischen etwa 30° und etwa 90°. Grundsätzlich kann der Schwenkwinkel auch größer als 90° sein.

Nach der Erfindung umfasst der Insertionskopf einen von der Basis abragenden Griff mit einer ersten Griffkomponente und einer zweiten Griffkomponente, die relativ zu der ersten Griffkomponente und der Basis beweglich ist. Die bewegliche zweite Griffkomponente ist mit der Einführeinrichtung so gekoppelt, dass eine Bewegung der zweiten Griffkomponente eine Bewegung der Einführeinrichtung in die Einführposition bewirkt. Aufgrund der Ausstattung des Griffs mit einer beweglichen Griffkomponente kann die Bewegung der Einführeinrichtung allein durch Greifen und Betätigen des Griffs bewirkt werden. Der Griff bildet selbst das Widerlager für die bewegbare zweite Griffkomponente. In diesem Sinne wird hier das Widerlager bildende Teil des Griffs als erste Griffkomponente bezeichnet. Die zweite Griffkomponente kann beispielsweise als Druckknopf gebildet sein. Die erste Griffkomponente kann ein Gehäuse sein, aus dem solch ein Druckknopf herausragt. In ebenfalls bevorzugten Ausführungen bilden die beiden Griffkomponenten erst gemeinsam den Griff, beispielsweise die Hälften eines insgesamt zweiteiligen Griffs.

Für die automatisierte Platzierung mittels des erfindungsgemäßen Inserters weist der Insertionskopf, vorzugsweise dessen Griff, eine Haltestruktur auf, die mit der Halteeinrichtung des Inserters in dem Halteeingriff ist. Falls der Griff wie bevorzugt eine mit der Basis unbeweglich verbundene Griffkomponente und eine relativ zu dieser Griffkomponente bewegliche Griffkomponente aufweist, bildet vorzugsweise die unbewegliche Griffkomponente die Haltestruktur. Die Haltestruktur kann an dem Griff oder der unbeweglichen Griffkomponente insbesondere in einem Stück angeformt sein, vorzugsweise ist sie mit dem Griff oder der unbeweglichen Griffkomponente zumindest steif verbunden.

Die bewegliche Griffkomponente bildet vorzugsweise das vorstehend in Bezug auf das System aus Insertionskopf und Inserter genannte Empfangsglied, wird jedoch nachfolgend als bewegliche Griffkomponente bezeichnet, solange besonders bevorzugte Ausführungen des Insertionskopfs beschrieben werden. Grundsätzlich muss das Empfangsglied des Systems jedoch keine Griffkomponente sein, sondern kann ausschließlich der Kopplung zwischen dem Aktivierungsglied und der Einführeinrichtung dienen.

In bevorzugten Ausführungen ist die bewegliche zweite Griffkomponente zumindest im Wesentlichen parallel zu der Unterseite der Basis bewegbar. Insbesondere kann es sich um eine Linearbeweglichkeit handeln. Alternativ zu einer reinen Translationsbeweglichkeit kann die zweite Komponente beispielsweise schwenkbeweglich angebracht sein.

Die erste Griffkomponente ist mit der Basis vorzugsweise nicht beweglich verbunden. Grundsätzlich wäre es jedoch auch denkbar, dass beide Griffkomponenten relativ zu der Basis beweglich mit dieser verbunden sind.

Für die Übertragung der Bewegung der zweiten Griffkomponente auf die Einführeinrichtung kann eine steife Kopplung vorgesehen sein, d.h. die zweite Griffkomponente und die Einfuhreinrichtung können steif miteinander verbunden sein, worunter auch eine Urformung in einem Stück verstanden wird. Eine steife Kopplung kann beispielsweise im Falle einer bevorzugten Schwenkbeweglichkeit der Einführeinrichtung ohne weiteres dann verwirklicht werden, wenn auch die zweite Griffkomponente schwenkbeweglich ist. Gegenüber dem Insertionskopf der deutschen Patentanmeldung Nr. 10 2004 039 408.3 weist der erfindungsgemäße Insertionskopf dennoch den Vorteil auf, dass die erste Griffkomponente dem Benutzer als Widerlager dienen kann und nicht das Gewebe über die Basis die für die Schwenkbewegung aufzubringende Kraft aufnehmen muss, vielmehr wird diese Kraft durch das Halten der ersten Griffkomponente vom Benutzer aufgenommen. Verfügt der Benutzer über einen Inserter, insbesondere den erfindungsgemäßen, mit dem der Insertionskopf auf dem Gewebe platziert und dabei die Einführrichtung eingeführt wird, nimmt ein derartiger Inserter diese Kraft auf.

In bevorzugten Ausführungen sind die bewegliche zweite Griffkomponente und die Einführeinrichtung über ein Getriebe miteinander gekoppelt. Eine derartige Kopplung hat den Vorteil, dass die Beweglichkeit der Griffkomponente nicht der Beweglichkeit der Einführeinrichtung entsprechen muss, sondern beide Beweglichkeiten jeweils einzeln für sich optimal gestaltet werden können. So kann die Einführeinrichtung insbesondere schwenkbeweglich und die zweite Griffkomponente translatorisch beweglich und dabei vorzugsweise linear geführt sein. Im Falle einer Schwenkbeweglichkeit auch der zweiten Griffkomponente kann deren Schwenkachse eine andere als die der Einführeinrichtung sein. Während die Einführeinrichtung vorzugsweise um eine zu der Unterseite der Basis zumindest im wesentlichen parallelen Rotationsachse schwenkbar ist, was im übrigen für sämtliche Ausführungsformen der zweiten Griffkomponente gilt, kann eine schwenkbare zweite Griffkomponente um eine zur Unterseite zumindest im wesentlichen rechtwinkligen Rotationsachse schwenkbeweglich sein. Ein Getriebe kann aber auch dann von Vorteil sein, wenn im Falle einer schwenkbeweglichen zweiten Griffkomponente deren Rotationsachse von der Rotationsachse der schwenkbeweglichen Einführeinrichtung parallel beabstandet ist. In solch einem Fall kann der Schwenkwinkel der zweiten Griffkomponente mittels eines Getriebes untersetzt oder vorzugsweise übersetzt auf die Einführeinrichtung übertragen werden.

Eine bevorzugte getriebetechnische Kopplung umfasst ein Zahnrad und eine Zahnstange, die miteinander in einem Zahneingriff sind und bei Bewegung der zweiten Griffkomponente miteinander kämmen. Die Zahnstange ist vorzugsweise mit der zweiten Griffkomponente verbunden, so dass eine Bewegung der zweiten Griffkomponente in Längsrichtung der Zahnstange in eine Drehbewegung des in diesem Fall mit der Einführeinrichtung verbundenen Zahnrads übertragen wird. Bei entsprechend feiner Verzahnung bzw. Zahnteilung kann ein vergleichsweise kurzer Hub der zweiten Griffkomponente in eine Drehbewegung des Zahnrads über einen beträchtlichen Teil einer vollen Umdrehung, vorzugsweise in eine viertel Umdrehung, des Zahnrads, übertragen werden. Vorteilhafterweise ist die bewegliche zweite Griffkomponente mit der Zahnstange in einem Stück geformt.

Der Griff ist in bevorzugten Ausführungen lösbar mit der Basis verbunden. Bevorzugt löst sich diese Verbindung bei der Bewegung der zweiten Griffkomponente, d.h. bei der Bewegung der Einführeinrichtung in die Einführposition, automatisch. Alternativ wäre es grundsätzlich jedoch ebenfalls denkbar, den Griff oder die Basis mit einer weiteren beweglichen Komponente auszustatten, durch deren Betätigung die Verbindung mit der Basis gelöst wird. Die Verbindung zwischen dem Griff und der Basis kann zwar durch einen reinen Reibschluss hergestellt sein, bevorzugter beruht sie jedoch ausschließlich auf einem Formschluss oder einer Kombination aus Form- und Reibschluss. Um die Verbindung zu schaffen, sind die Basis und der Griff, vorzugsweise dessen erste Griffkomponente, je mit wenigstens einem Verbindungselement ausgestattet, die bei bestehender Verbindung miteinander in einem Eingriff sind. Um die Verbindung lösen zu können, ist vorzugsweise wenigstens eines der Verbindungselemente gegen eine rückstellende Elastizitätskraft aus dem Eingriff bewegbar. In bevorzugten Ausführungen dient die bewegliche zweite Griffkomponente nicht nur der Überführung der Einführeinrichtung in die Einführposition, sondern auch dem Lösen der Verbindung, indem die zweite Griffkomponente bei ihrer Bewegung durch Kontakt, vorzugsweise Gleitkontakt, eines der Verbindungselemente gegen die Elastizitätskraft aus dem Eingriff bewegt, beispielsweise elastisch abbiegt. Obgleich weniger bevorzugt, kann die erste Griffkomponente jedoch alternativ in einem Stück mit der Basis geformt oder unlösbar an der Basis befestigt sein. In solch einer Ausführung sollte sie jedoch möglichst kurz sein.

In der Schutzposition ist zumindest das freie Ende der Einfuhreinrichtung, bevorzugter die gesamte Einführeinrichtung, in einer Aufnahme aufgenommen, die entweder die Basis oder der Griff bildet. Falls der Griff die Aufnahme bildet, kann die Basis eine Teilaufnahme bilden, die in der vom Griff gebildeten Aufnahme aufgenommen ist, solange im Falle des bevorzugt lösbaren Griffs dieser mit der Basis verbunden ist.

Falls die Einführeinrichtung von Hause aus flexibel ist, d.h. nicht erst durch Wechselwirkung mit dem Gewebe flexibel wird, wird sie vorzugsweise mittels einer Einstecheinrichtung stabilisiert, um zu verhindern, dass die Einführeinrichtung bei dem Einführen in das Gewebe knickt. Die Einstecheinrichtung kann insbesondere als dünne Einstechnadel gebildet sein. Wenn die Einführeinrichtung in das Gewebe eingeführt worden ist, wird die Einstecheinrichtung vorteilhafterweise entfernt. Das Entfernen einer derartigen Einstecheinrichtung wird vorzugsweise ebenfalls mit dem Griff bewerkstelligt. Falls die Einstecheinrichtung wie bevorzugt in der Schutzposition noch nicht mit dem Griff verbunden ist, verbindet sie sie bei der Bewegung in die Einführposition vorzugsweise automatisch mit dem Griff. Hierfür kann an ihrem von dem freien Ende der Einführeinrichtung abgewandten Ende ein Verbindungselement, vorzugsweise ein Schnappelement, vorgesehen sein, das zugleich mit dem Abschluss der Bewegung in die Einführposition oder kurz zuvor mit einem Verbindungsgegenelement des Griffs in einen Verbindungseingriff gelangt. Die Verbindung kann zwar grundsätzlich rein reibschlüssig sein, vorzugsweise umfasst sie jedoch einen Formschluss zumindest. Das Verbindungselement der Einstecheinrichtung kann mit dem Verbindungsgegenelement insbesondere eine Schnappverbindung bilden. Grundsätzlich genügt für eine formschlüssige Verbindung auch nur ein einfacher Hintergriff bezüglich der Richtung, in die der Griff von der Basis entfernt werden soll; ein elastischer Schnappeingriff ist daher nicht unumgänglich erforderlich.

Der Insertionskopf ist für eine medizinische oder pharmazeutische, einschließlich kosmetische, Anwendung vorgesehen. Zumindest die Unterseite der Basis ist gewebeverträglich gebildet. Der Insertionskopf ist vorzugsweise Bestandteil eines Infusionssets für die Verabreichung von Insulin, eines Schmerzmittels oder eines anderen per Infusion verabreichbaren Medikaments. Anstatt für eine Medikamentenverabreichung oder grundsätzlich auch eines anderen verabreichbaren Produkts kann der Insertionskopf auch zu Diagnosezwecken dienen. In solchen Applikationen kann die Einführeinrichtung Träger eines Sensors zum Messen von beispielsweise der Glukosekonzentration in einer Körperflüssigkeit oder einer anderen physikalischen und/oder biochemischen Größe dienen, die für den Gesundheitszustand eines Patienten maßgeblich ist oder sein kann. Der Insertionskopf kann zu Diagnosezwecken auch als Perfusionsvorrichtung gebildet sein. In solch einer Ausbildung wird die Einführeinrichtung nach dem Einbringen in das Gewebe von einer Spülflüssigkeit durchströmt, die ein oder mehrere bestimmte Inhaltsstoffe der Körperflüssigkeit bei dem Durchströmen aufnimmt, um die mit dem betreffenden Inhaltsstoff oder den mehreren Inhaltsstoffen angereicherte Spülflüssigkeit zu analysieren. Schließlich kann der Insertionskopf eine Vorrichtung für die Verabreichung eines Produkts und eine Diagnoseeinrichtung in Kombination bilden. Die Einführeinrichtung kann für die Zuführung eines Produkts, das insbesondere ein Medikament oder eine Spülflüssigkeit sein kann, oder die Abführung einer Körperflüssigkeit oder nur eines oder mehrerer bestimmter Inhaltsstoffe einer Körperflüssigkeit gebildet sein, d.h. die Einführeinrichtung bildet in solch einer Applikation wenigstens einen Strömungsquerschnitt. Die Einführeinrichtung kann der Zu- und Abführung von Stoffen auch in Kombination dienen. Ist der Insertionskopf nur als Messvorrichtung gebildet, so kann sie auch nur dazu dienen, einen Sensor oder einen Teil eines Sensors zu platzieren, d.h. rein als mechanische Einbringeinrichtung. In einer Weiterbildung als Messvorrichtung kann sie über das mechanische Einbringen hinaus auch der Übertragung von Steuersignalen zu dem Sensor und/oder von Messsignalen von dem Sensor dienen. In kombinierten Applikationen kann sie schließlich über wenigstens einen Strömungsquerschnitt für den Stofftransport, d.h. eine Strömungsleitung, und wenigstens eine Signalleitung verfügen. Auf die Signalleitung kann verzichtet werden, wenn der Sensor für den drahtlosen Empfang von Steuersignalen und/oder das drahtlose Senden von Messsignalen eingerichtet ist. Schließlich kann die Einführeinrichtung auch zwei oder mehr Einführabschnitte aufweisen, die separat abragen. So kann ein erster Einführabschnitt dem Stofftransport in das Gewebe und ein anderer dem Stofftransport aus dem Gewebe oder nur dem Einbringen eines Sensors oder eines Teils eines Sensors dienen. Mit mehreren Einführabschnitten, die je einen Strömungsabschnitt aufweisen, können mit dem gleichen Insertionskopf auch unterschiedliche Stoffe verabreicht werden. Dies kann auch mit einer Einführeinrichtung verwirklicht werden, die mehrere, separate Strömungsquerschnitte in einem gemeinsamen Abschnitt bildet.

Bevorzugte Merkmale werden auch in den Unteransprüchen und deren Kombinationen offenbart.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: einen Insertionskopf eines ersten Ausführungsbeispiels mit in Schutzposition befindlicher Einführeinrichtung,
- Figur 2: den Insertionskopf mit in Einführposition befindlicher Einführeinrichtung,
- Figur 3: einen Griff des Insertionskopfs des ersten Ausführungsbeispiels,
- Figur 4: eine Basis des Insertionskopfs des ersten Ausführungsbeispiels mit in Einführposition befindlicher Einführeinrichtung,
- Figur 5: den Griff der Figur 3 in einer Ansicht
- Figur 6: die Basis mit Einführeinrichtung der Figur 4 in einer Ansicht,
- Figur 7: einen Insertionskopf eines zweiten Ausführungsbeispiels mit in Schutzposition befindlicher Einführeinrichtung,
- Figur 8: den Insertionskopf des zweiten Ausführungsbeispiels mit in Einführposition befindlicher Einführeinrichtung,
- Figur 9: einen Griff des Insertionskopfs des zweiten Ausführungsbeispiels,
- Figur 10: eine Basis des Insertionskopfs des zweiten Ausführungsbeispiels mit in Einführposition befindlicher Einführeinrichtung,
- Figur 11: ein System aus einem Insertionskopf und einem Inserter eines ersten Ausführungsbeispiels vor einer Aktivierung,
- Figur 12: das System des ersten Ausführungsbeispiels nach der Aktivierung,
- Figur 13: ein System aus einem Insertionskopf und einem Inserter eines zweiten Ausführungsbeispiels vor einer Aktivierung des Insertionskopfs,
- Figur 14: das System des zweiten Ausführungsbeispiels nach der Aktivierung und
- Figur 15: das System des zweiten Ausführungsbeispiels nach einer Platzierung des Insertionskopfs auf einer Gewebeoberfläche.

Nachfolgend werden gleiche oder zumindest funktionsgleiche Bestandteile in der Regel mit gleichen oder vergleichbaren Bezugszeichen benannt, so dass eine mehrfache Beschreibung meist unterbleiben kann. Die Bestandteile der einzelnen Ausführungsformen können größtenteils miteinander ausgetauscht werden, d.h., kombiniert werden. Aus den nachfolgend beschriebenen Ausführungen gehen weitere Merkmale, Zielsetzungen sowie Vorteile der Erfindung hervor.

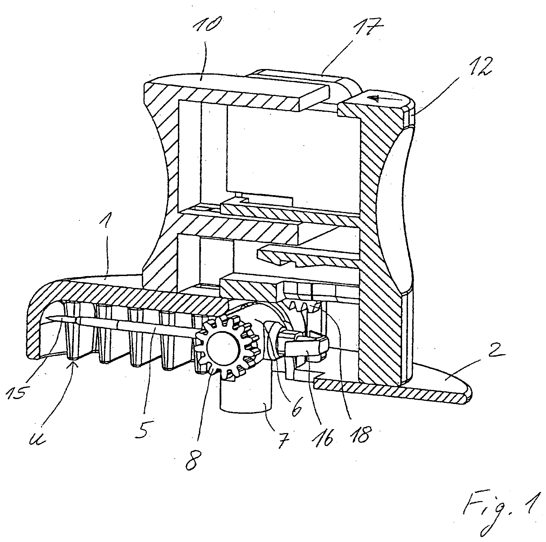

Figur 1 zeigt einen Insertionskopf eines ersten Ausführungsbeispiels in einem Längsschnitt. Der Insertionskopf umfasst eine Basis mit einem Aufnahmeteil bzw. einer Aufnahme 1 und einem Flachteil 2, die in einem Stück aus Kunststoff geformt sind. Die Basis 1, 2 ist mit ihrer Unterseite U auf organischem Gewebe platzierbar. Der Insertionskopf umfasst des Weiteren einen zweiteiligen Griff mit einer ersten Griffkomponente 10 und einer zweiten Griffkomponente 12. Die Griffkomponente 10 ist mit der Basis unbeweglich, aber lösbar verbunden. Die Griffkomponente 12 ist an der Griffkomponente 10 beweglich gehalten, wobei die Griffkomponente 12 sowohl relativ zu der Griffkomponente 10 als auch zu der Basis 1, 2 linear verschiebbar ist. Die Achse der Bewegbarkeit der Griffkomponente 12 weist parallel zu einer Unterseite U der Basis 1, 2. Die Richtung der Bewegbarkeit ist auf der Oberseite der Griffkomponente 12 mit einem Pfeil angedeutet.

Die Basis 1, 2 lagert eine Einführeinrichtung 5 schwenkbeweglich um eine zur Unterseite U parallele Rotationsachse. Die Einführeinrichtung 5 ist lang gestreckt. Im Ausführungsbeispiel ist sie als flexible Kanüle gebildet. Die Einführeinrichtung 5 wird von einer Einstecheinrichtung 15 durchragt, die als dünne Nadel gebildet ist, deren Biegesteifigkeit ausreicht, um die Einstecheinrichtung 15 gemeinsam mit der umgebenden, sich anschmiegenden Einführeinrichtung 5 durch die Hautoberfläche in subkutanes Gewebe einzustechen und dadurch die Einführeinrichtung 5 einzuführen. In bevorzugten Ausführungen ist an der Unterseite U ein Klebepad für eine Fixierung des Insertionskopfs auf dem Gewebe, vorzugsweise der Hautoberfläche, angebracht.

Für die Schwenkbeweglichkeit der Einführeinrichtung 5 und damit gemeinsam der Einstecheinrichtung 15 sorgt ein Gelenkelement 6, das eine Welle eines Drehgelenks mit der Rotationsachse als Gelenkachse bildet. Die Basis 1, 2 bildet das andere Gelenkelement des Drehgelenks in Form einer Buchse oder gegebenenfalls auch eines offenen Lagerauges. Auf der Rotationsachse des Gelenks ist zu beiden Seiten des Gelenkelements 6 je ein außenverzahntes Zahnrad 8 angeordnet und drehsteif mit dem Gelenkelement 6 verbunden, beispielsweise in einem Stück geformt. Das eine der beiden Zahnräder 8 ist in Figur 1 erkennbar. Das andere sitzt auf der gegenüberliegenden Seite des Gelenkelements 6 und wird von dem Aufnahmeteil 1 der Basis 1, 2 verdeckt. Die Einstecheinrichtung 15 durchragt das Gelenkelement 6. An das Gelenkelement 6 schließt sich eine Zuführung 7 für eine Medikamentenflüssigkeit, beispielsweise Insulin, an. Die Zuführung 7 ragt in etwa rechtwinklig zur Einführeinrichtung 5 von dem Gelenkelement 6 ab. Das Gelenkelement 6 bildet mit der Zuführung 7, den Zahnrädern 8, der Einführeinrichtung 5 und der Einstecheinrichtung 15 eine in Bezug auf die Drehbewegung des Gelenkelements 6 und der Zahnräder 8 und der Schwenkbewegung der genannten weiteren Komponenten eine Einheit.

Die bewegliche Griffkomponente 12 ist mit zwei Zahnstangen 18 versehen, die je mit einem der Zahnräder 8 in Zahneingriff sind. Von den beiden Zahnstangen 18 ist nur die mit dem verdeckten Zahnrad zusammenwirkende erkennbar. Eine ebensolche Zahnstange 18 wirkt mit dem erkennbaren Zahnrad 8 zusammen. Im Falle einer Verschiebung der Griffkomponente 12 in die durch den Richtungspfeil angedeutete Richtung, wobei die erste Griffkomponente 10 bei dieser Bewegung die Griffkomponente 12 führt, kämmen die beiden Zahnstangen 18 mit den beiden Zahnrädern 8, so dass die Verschiebebewegung der Griffkomponente 12 in eine Drehbewegung des Gelenkelements 6 und eine Schwenkbewegung der Einführeinrichtung 5 und der Einstecheinrichtung 15 sowie der Zuführung 7 übertragen wird.

Die Schwenkbewegung überführt die Einführeinrichtung 5 aus deren in Figur 1 gezeigten Schutzposition in eine Einführposition. In der Schutzposition weisen die Einführeinrichtung 5 und die Einstecheinrichtung 15 zumindest im Wesentlichen parallel zu der Unterseite U der Basis 1, 2. Die Einführeinrichtung 5 und der in die gleiche Richtung über das Gelenkelement 6 hinausstehende Abschnitt der Einstecheinrichtung 15 sind in der gemeinsamen Schutzposition in einem von dem Aufnahmeteil bzw. der Aufnahme 1 mit Ausnahme der Unterseite U umschlossenen Hohlraum aufgenommen. Mit in Schutzposition befindlicher Einführeinrichtung 5 ist gewährleistet, dass der Benutzer sich nicht an der Einstecheinrichtung 15 verletzen und umgekehrt die Einführeinrichtung 5 und die Einstecheinrichtung 15 nicht durch unachtsame Handhabung beschädigt werden können. Die Aufnahme 1 bildet bevorzugt auch einen Sichtschutz, so dass der Benutzer die Einstecheinrichtung 15 von der Oberseite des Insertionskopfs und auch bei seitlichem Blickwinkel nicht erkennen kann. Ein an der Unterseite bevorzugterweise angebrachtes Klebepad ist mit einem Durchtrittsschlitz für die Einführeinrichtung 5 und die Einstecheinrichtung 15 versehen.

Die Zahnstangen 18 sind jeweils an einer der Unterseite U zugewandten Unterseite zweier biegesteifer Zungen geformt, die von einem Seitenteil der Griffkomponente 12 in Bewegungsrichtung vorragen. Über die beiden die Zahnstangen 18 bildenden Zungen hinaus ragt von dem Seitenteil der Griffkomponente 12 wenigstens eine weitere Zunge in Bewegungsrichtung vor, die der Linearführung der beweglichen Griffkomponente 12 an einer von der Griffkomponente 10 gebildeten Führung dient.

Um die Einführeinrichtung 5 in das Körpergewebe bis unter oder gegebenenfalls nur in die Haut einzuführen, greift der Benutzer den Insertionskopf am Griff zwischen Daumen und Zeigefinger. Die Griffkomponenten 10 und 12 sind je mit einer entsprechend geformten, seitlichen Einbuchtung versehen. Durch Zusammendrücken der Griffkomponenten 10 und 12 wird die bewegliche Griffkomponente 12 bis gegen einen von der ersten Griffkomponente 10 gebildeten Anschlag gedrückt. Bei dieser Bewegung kämmen die beiden Zahnstangen 18 mit den Zahnrädern 8, so dass die Translationsbewegung der Griffkomponente 12 in eine Drehbewegung des Gelenkelements 6 und somit in eine Schwenkbewegung der Einführeinrichtung 5 und der Einstecheinrichtung 15 übertragen wird. Der Weg der Griffkomponente 12, der Durchmesser der Zahnräder 8 und die Feinheit der Verzahnungen sind so gewählt, dass eine Bewegung der Griffkomponente 12 um wenige Millimeter, beispielsweise 4 oder 5 mm, eine Schwenkbewegung der Einführeinrichtung und der Einstecheinrichtung 15 um einen Schwenkwinkel von zumindest im wesentlichen 90° in eine Einführposition bewirkt, in der die Einführeinrichtung 5 und die Einstecheinrichtung 15 über die Unterseite U der Basis 1, 2 zumindest in etwa rechtwinklig vorragen.

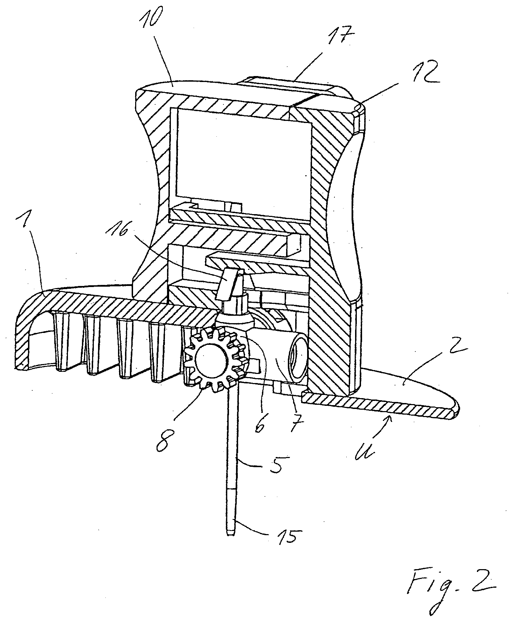

Figur 2 zeigt den Insertionskopf mit der in Einführposition befindlichen Einführ- und Einstecheinrichtung 5, 15.

Die Einstecheinrichtung 15 hat sich am Ende der Schwenkbewegung mit dem Griff 10, 12 verbunden, im Ausführungsbeispiel mit der beweglichen Griffkomponente 12. In der Schutzposition (Figur 1) besteht zwischen der Einstecheinrichtung 15 und dem Griff 10, 12 kein Kontakt, so dass die Einstecheinrichtung 15 gemeinsam mit der Einführeinrichtung 5 frei schwenken kann. Zur Herstellung der Verbindung ist an einem in der Einführposition proximalen Ende der Einstecheinrichtung 15, mit dem die Einstecheinrichtung 15 über das Gelenkelement 6 hinaussteht, ein Verbindungselement 16 angeordnet, im Ausführungsbeispiel befestigt. Das Verbindungselement 16 weist einen oder zwei abstehende Flügel auf, mit denen es ein Verbindungsgegenelement der Griffkomponente 12 in Bezug auf die Längsrichtung der Einstecheinrichtung 15 hintergreift. Das Verbindungsgegenelement der beweglichen Griffkomponente 12 ist als entsprechend hintergreifbare Schulterfläche gebildet.

Für die Platzierung des Insertionskopfs auf einer Gewebeoberfläche und der Einführung der Einführeinrichtung 5 in das Gewebe hält der Benutzer den Insertionskopf am Griff 10, 12 und bewegt ihn auf die Gewebeoberfläche zu. Dabei durchsticht die Einstecheinrichtung 15 die Gewebeoberfläche, vorzugsweise die menschliche Haut, und dringt in die Haut ein. Die sich anschmiegende Einführeinrichtung 5 dringt gemeinsam mit der Einstecheinrichtung 15 ein, bis der Insertionskopf mit seiner Unterseite U auf der Gewebeoberfläche aufsetzt und sich dabei adhäsiv auf der Hautoberfläche fixiert, vorzugsweise mittels eines Klebepads. Für die Verabreichung des Medikaments wird die Einstecheinrichtung 15 entfernt und die Zuführung 7 über einen mit der Zuführung 7 zusammenwirkenden Konnektor mit einem Medikamentenreservoir, vorzugsweise einer Medikamentenpumpe, verbunden. Um dies zu ermöglichen, wird zuvor der Griff 10, 12 von der Basis 1, 2 gelöst. Das Lösen ist nur nach dem Zusammendrücken bzw. Zusammenschieben der Griffkomponenten 10 und 12 möglich. Allerdings löst sich die Verbindung bei der Bewegung der Griffkomponente 12 automatisch, so dass der Griff 10, 12 in die proximale Richtung, in Figur 2 nach oben, abgezogen werden kann. Bei der geraden Abziehbewegung gleitet die Einstecheinrichtung 15 durch die Einführeinrichtung 5 und das Gelenkelement 6 und gibt dadurch den Strömungsquerschnitt der Einführeinrichtung 5 frei, so dass der Strömungsquerschnitt nach Herausziehen der Einstecheinrichtung 15 auch gleichzeitig mit der Zuführung 7 fluidisch verbunden ist. Diesbezüglich kann der Insertionskopf wie beispielsweise in der DE 198 21 723 C1 und der DE 10 2004 039 408.3 beschrieben ausgebildet sein.

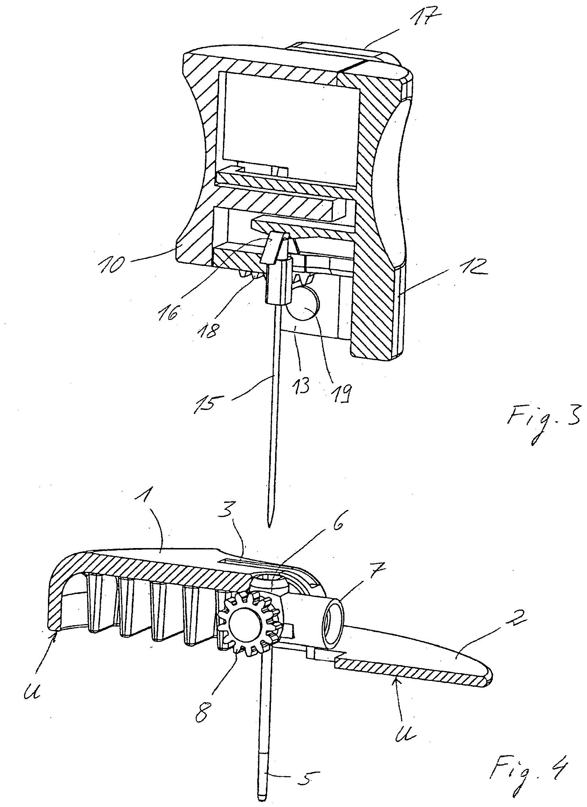

Die Figuren 3 und 4 zeigen die beiden voneinander gelösten Teile des Insertionskopfs, nämlich die Basis 1, 2 mit der Einführeinrichtung 5 einerseits und den Griff 10, 12 mit der Einstecheinrichtung 15 andererseits, in zueinander ausgerichteter Position, in der die Längsachse der Einführeinrichtung 5 und die Längsachse der Einstecheinrichtung 15 miteinander fluchten. In Figur 3 ist auch eine Ausnehmung 3 in der Basis 1, 2 erkennbar, in die im verbundenen Zustand bei Bewegung der Griffkomponente 12 eine der beiden Zahnstangen 18 einfährt und dabei mit dem in der Ausnehmung 3 angeordneten Zahnrad 8 kämmt. Die Ausnehmung 3, ist schlitzförmig. Ferner ist ein Verbindungselement 19 des Griffs 10, 12 erkennbar, das im verbundenen Zustand formschlüssig in ein Verbindungsgegenelement der Basis 1, 2 eingreift und so den Griff 10, 12 an der Basis 1, 2 hält und in Kombination mit Kontaktflächen des Griffs 10, 12 und der Basis 1, 2 relativ zu der Basis 1, 2 fixiert. Das Verbindungselement 19 ragt stummelartig von einer im distalen Bereich von der Griffkomponente 10 abragenden, elastischen Lasche 13 in eine zur Unterseite U der Basis 1, 2 parallel weisende Richtung vor und im verbundenen Zustand in eine Ausnehmung der Basis 1, 2 hinein, beispielsweise ein zum Verbindungselement 19 kongruent geformtes Loch, so dass eine Bewegung des Griffs 10, 12 in Längsrichtung des Einstechabschnitts 15 bei bestehender Verbindung verhindert wird. Um diese Verbindung zu lösen, ragt von dem Seitenteil der beweglichen Griffkomponente 12 eine nicht dargestellte weitere Zunge vor, die bei der Bewegung der Griffkomponente 12 zwischen die Basis 1, 2 und die das Verbindungselement 19 tragende Lasche 13 fährt und die Lasche 13 von der Basis 1, 2 ein Stück weit abbiegt, das jedoch ausreicht, die formschlüssige Verbindung zwischen dem Verbindungselement 19 und dem Verbindungsgegenelement zu lösen, so dass der Griff 10, 12 mit der Einstecheinrichtung 15 in deren Längsrichtung von der Basis 1, 2 abgezogen werden kann.

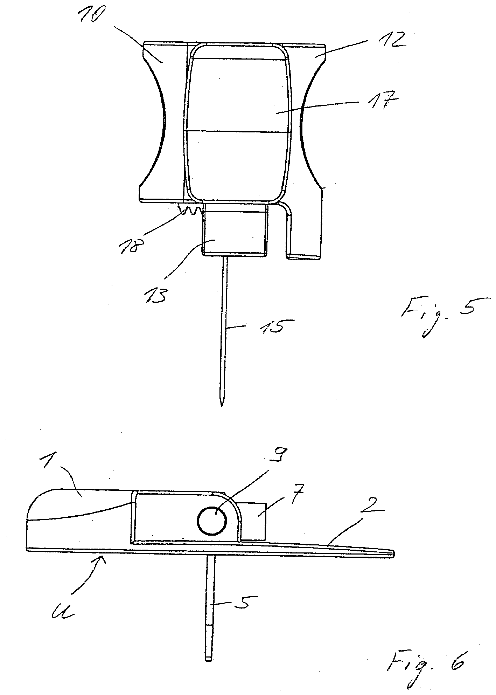

Die Figuren 5 und 6 zeigen nochmals die voneinander gelösten Teile des Insertionskopfs in einer Ansicht auf die in den Figuren 1 bis 4 abgewandte Rückseite. In dieser Ansicht ist in Figur 6 insbesondere das Verbindungsgegenelement 9 der Basis 1, 2 erkennbar, das im verbundenen Zustand, d.h. im Eingriff mit dem Verbindungselement 19, den Griff 10, 12 an der Basis 1, 2 hält.

Die in den Figuren 4 und 6 einzeln dargestellte Basis 1, 2, die Träger der Einführeinrichtung 5 und der damit eine Schwenkeinheit bildenden Teile 6, 7 und 8 ist, verbleibt auf der Gewebeoberfläche und ist in diesem Sinne ein Verbleibteil. Der Griff 10, 12 hingegen, der nun als Träger für die Einstecheinrichtung 15 dient, wird entsorgt oder gegebenenfalls wieder von der Einstecheinrichtung 15 gelöst und einer erneuten Verwendung zugeführt, während die Einstecheinrichtung 15 entsorgt wird. Das Verbleibteil 1-9 kann somit vorteilhaft flach sein und stört nicht, wenn es unter der Kleidung getragen wird. Die Flexibilität der Einführeinrichtung 5 ist derart, dass die Einführeinrichtung 5 im eingeführten Zustand als nicht störend empfunden wird, aber doch ausreichend stabil ist, um eine Versorgung mit dem Medikament sicher zu gewährleisten.

Der Griff 10, 12 kann auch in solchen Ausführungen des Insertionskopfs verwendet werden, in denen die Einführeinrichtung nicht wie die Einführeinrichtung 5 von Hause aus flexibel ist, sondern ohne Fremdstabilisierung für das Einstechen ausreichend biegesteif ist. In derartigen Ausführungen kann die zusätzliche Einstecheinrichtung 15 entfallen. Der Griff 10, 12 dient in derartigen Ausführungen ausschließlich der Handhabung des Insertionskopfs, nicht jedoch als Träger einer stabilisierenden Einstecheinrichtung 15. Eine derart modifizierte Einführeinrichtung 5 kann insbesondere als Einstechkanüle mit einem Hohlquerschnitt oder als Einstechnadel mit einem vollen Querschnitt und einem oder mehreren Strömungskanälen am äußeren Umfang gebildet sein, die nach der Einführung durch Wechselwirkung mit dem Gewebe flexibler wird.

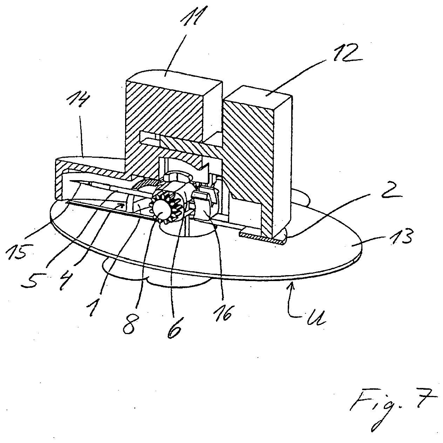

Die Figuren 7 bis 10 zeigen ein zweites Ausführungsbeispiel eines Insertionskopfs. Von den nachfolgend beschriebenen Unterschieden abgesehen entspricht der Insertionskopf des zweiten Ausführungsbeispiels dem Insertionskopf des ersten Ausführungsbeispiels.

So ist beispielhaft ein an der Unterseite U befestigtes Klebepad dargestellt, wie es auch an dem Insertionskopf des ersten Ausführungsbeispiels angebracht sein könnte.

Gegenüber dem ersten Ausführungsbeispiel sind die Aufnahme 1 und die erste Griffkomponente 11 modifiziert. Im Unterschied zum ersten Ausführungsbeispiel sind die Einführeinrichtung 5 und die Einstecheinrichtung 15 nur über einen kurzen Abschnitt in der vom Aufnahmeteil gebildeten Aufnahme 1 aufgenommen. Im zweiten Ausführungsbeispiel bildet der Griff, genauer gesagt dessen Griffkomponente 11, eine Aufnahme 14 für die Einführeinrichtung 5 und die Einstecheinrichtung 15. Die Aufnahme 1 ist seitlich mit einer zur Unterseite U offenen Ausnehmung 4 in Form eines Schlitzes versehen, durch welche die Einführeinrichtung 5 und die Einstecheinrichtung 15 in der Schutzposition aus der Aufnahme 1 hinausragen. Die Aufnahme 1 ist ihrerseits in der Aufnahme 14 aufgenommen. Die Aufnahme 14 ist zur Unterseite U hin offen, umschließt aber ansonsten die Einführeinrichtung 5 und die Einstecheinrichtung 15, vorzugsweise blickdicht.

Um die Einführeinrichtung 5 und die Einstecheinrichtung 15 aus der Schutzposition in die Einführposition zu schwenken, führt der Benutzer die anhand des ersten Ausführungsbeispiels beschriebenen Handgriffe durch, d.h. er drückt die bewegliche Griffkomponente 12 gegen die modifizierte Griffkomponente 11. Bei dem kämmenden Zahneingriff schwenken die Einführeinrichtung 5 und Einstecheinrichtung 15 in der Ausnehmung 4 aus der Aufnahme 1 und insbesondere aus der Aufnahme 14 in die Einführposition.

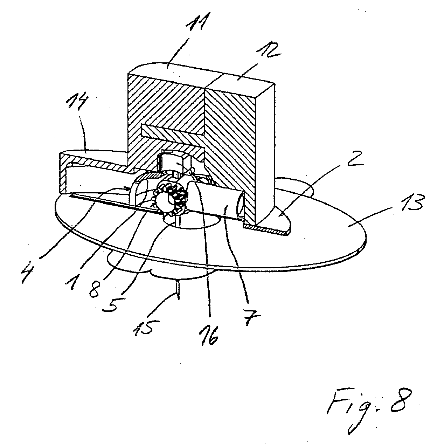

Figur 8 zeigt den Insertionskopf des zweiten Ausführungsbeispiels mit in Einführposition befindlicher Einführeinrichtung 5 und Einstecheinrichtung 15.

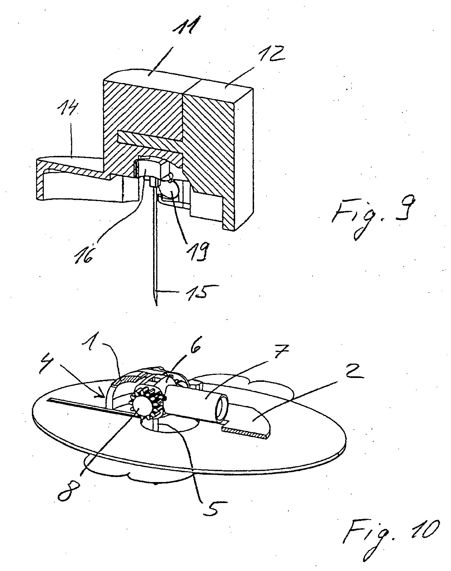

Die Figuren 9 und 10 entsprechend bezüglich des zweiten Ausführungsbeispiels den Figuren 3 und 4 zum ersten Ausführungsbeispiels. Wie jedoch nicht zuletzt der Blick auf Figur 10 zeigt, ist die Basis 1, 2 des zweiten Ausführungsbeispiels gegenüber der Basis 1, 2 des ersten Ausführungsbeispiels vorteilhaft verkürzt, da nicht mehr sie, sondern der Griff 11, 12 für die Einführeinrichtung 5 und die Einstecheinrichtung 15 in deren Schutzposition die Schutzfunktion übernimmt.

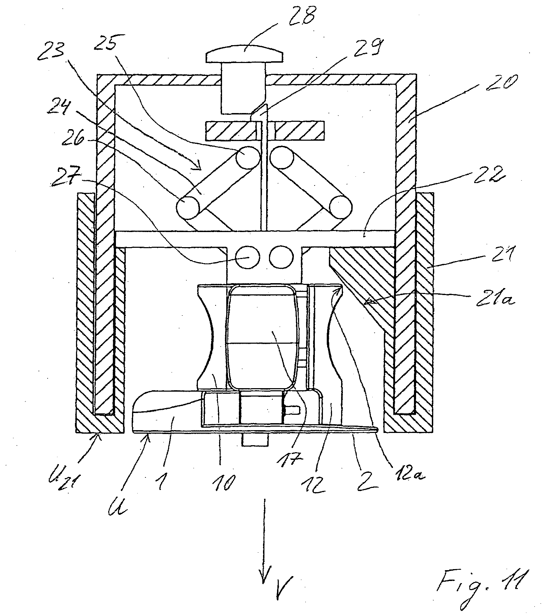

Figur 11 zeigt ein System eines ersten Ausführungsbeispiels bestehend aus dem Insertionskopf des ersten Ausführungsbeispiels und einem Inserter, welcher der Platzierung des Insertionskopfs auf dem Gewebe dient, so dass der Benutzer den Insertionskopf bei der Platzierung nicht zwischen den Fingern greifen muss. Insbesondere hält der Benutzer bei der Überführung der Einführeinrichtung 5 in die Einführposition den Insertionskopf nicht am Griff. Diese Aktivierung des Insertionskopfs wird mit Hilfe des Inserters vorgenommen. Der Benutzer wird daher noch sicherer vor Stichverletzungen und die Einführeinrichtung 5 sowie die Einstecheinrichtung 15 werden noch sicherer vor Beschädigungen durch unachtsame Handhabung geschützt, nämlich durch den Inserter.

Der Inserter weist ein Insertergehäuse 20 auf, das als Hülsenteil mit einem Boden gebildet ist und von außen betrachtet im Wesentlichen die Form eines Topfs aufweist. Das Insertergehäuse 20 nimmt eine Halteeinrichtung und einen Antrieb für den Insertionskopf auf. Die Halteeinrichtung umfasst eine Haltefeder, beispielsweise eine Blattfeder, die den Insertionskopf in der in Figur 11 gezeigten Ausgangsposition relativ zu dem Insertergehäuse 20 hält. In dem Halteeingriff hintergreift die Haltefeder eine an dem Griff 10, 12 geformte Haltestruktur 17, die in den Figuren 1, 2, 3 und 5 erkennbar ist. Der Halteeingriff ist gegen die rückstellende Elastizitätskraft der Haltefeder lösbar.

Der Antrieb umfasst ein Vortriebselement 22, das in und gegen eine Vortriebsrichtung V linear beweglich in dem Insertergehäuse 20 angeordnet ist. Die Vortriebsrichtung V fällt mit einer zentralen Längsachse des Insertergehäuses 20 zusammen. Der Antrieb umfasst ferner einen Krafterzeuger 23, der in die Vortriebsrichtung V auf das Vortriebselement 22 wirkt. Der Krafterzeuger 23 umfasst zwei Paare von gelenkig miteinander verbundenen Schenkeln 24, wobei die beiden Paare von Schenkeln 24 symmetrisch bezüglich der zentralen Längsachse, d. h. symmetrisch zur Vortriebsrichtung V, des Insertergehäuses 20 angeordnet sind. Jedes der Schenkelpaare ist in einem zum Insertergehäuse 20 ortsfesten Drehgelenk 25 aufgehängt. Die beiden Schenkel 24 des jeweiligen Schenkelpaars sind in einem freien Drehgelenk 26 miteinander drehbeweglich verbunden. Ferner ist der von dem ortsfesten Gelenk 25 abgewandte Schenkel 24 jeweils in einem Drehgelenk 27 mit dem Vortriebselement 22 verbunden. Nicht dargestellte Federn oder gegebenenfalls auch nur eine Feder spannt diese Schenkel-Gelenk-Vortriebselement-Anordnung in die Vortriebsrichtung V. Die Anordnung aus Schenkeln 24 und Gelenken 25, 26 und 27 führt das Vortriebselement 22; zusätzlich oder stattdessen könnte die Mantelinnenfläche des Insertionsgehäuses 23 das Vortriebselement 22 führen. Ferner ist ein Blockierglied 29 vorgesehen, das mit dem Insertergehäuse 20 in einem Blockiereingriff ist, der eine Vortriebsbewegung des Vortriebselements 22 verhindert. Das Blockierglied 29 kann den Blockiereingriff mit der von dem Insertergehäuse 20 gebildeten Hüllstruktur oder ebenso mit einer damit in Bezug auf die Vortriebsrichtung V fest verbundenen sonstigen Struktur bilden. Der Blockiereingriff ist durch Betätigung eines druckknopfartigen Auslösers 28 lösbar.

Der Inserter umfasst ferner ein Aktivierungsglied 21, das mit dem Insertergehäuse 20 in und gegen die Vortriebsrichtung. V beweglich verbunden ist. Das Aktivierungsglied 21 bildet in Bezug auf das Insertergehäuse 20 eine Muffe, so dass insgesamt ein zweiteiliges, teleskopierbares Insertergehäuse mit Gehäuseteilen 20 und 21 erhalten wird. Der Unterscheidung in funktionaler Hinsicht wegen wird das Gehäuseteil 21 jedoch weiterhin als Aktivierungsglied bezeichnet. Das Aktivierungsglied 21 bildet die Unterseite U21 des Inserters, mit welcher der Inserter für die Platzierung des Insertionskopfs auf der Gewebeoberfläche aufsetzbar ist und vorzugsweise auch aufgesetzt wird. In der in Figur 11 vom Insertionskopf eingenommenen Ausgangsposition weisen die Unterseite U21 des Inserters und die Unterseite U des gehaltenen Insertionskopfs jeweils in die Vortriebsrichtung V, die für die beiden Unterseiten zumindest im Wesentlichen eine Flächennormale bildet.

Das Aktivierungsglied 21 umfasst ein äußeres Hülsenteil und ein inneres Hülsenteil, die an der Unterseite U21 miteinander verbunden sind und zwischen sich einen Ringspalt freilassen. Das Insertergehäuse 20 ragt in diesen Ringspalt und führt das Aktivierungsglied 21 gleitbeweglich.

In dem in Figur 11 gezeigten Zustand nimmt das Aktivierungsglied 21 relativ zu dem Insertergehäuse 20 eine eingefahrene Position ein und der Inserter weist eine in Vortriebsrichtung V gemessen kürzeste Länge auf. In diesem Zustand des Inserters wird der Insertionskopf eingesetzt, d. h. in den Halteeingriff mit der Halteeinrichtung des Inserters gebracht. Anstatt den Insertionskopf einzusetzen, kann der Inserter auch über den auf einer Auflage liegenden Insertionskopf gestülpt werden. Die Position und Geometrie der Halteeinrichtung ist so gewählt, dass der Halteeingriff bei dem Aufstülpen automatisch entsteht. Unmittelbar nach dem Aufnehmen des Insertionskopfs, beispielsweise durch Einsetzen, befindet sich die Einfiihreinrichtung 5 des Insertionskopfs in ihrer Schutzposition. In diesem Sinne ist der Insertionskopf noch inaktiv. Der Inserter ist mit Mitteln ausgestattet, nämlich dem Aktivierungsglied 21, durch deren Betätigung die Einführeinrichtung in die Einführposition bewegt und auf diese Weise der Insertionskopf aktiviert werden kann.

Für die Aktivierung bilden das Aktivierungsglied 21 und der Insertionskopf miteinander ein Gelenk, im Ausführungsbeispiel ein Kurvengelenk. Die beiden Gelenkelemente des Gelenks sind eine Führungskurve 21a, die das Aktivierungsglied 21 bildet, und ein von der beweglichen Griffkomponente 12 gebildetes Eingriffselement 12a. In der Kopplung, über die das Aktivierungsglied 21 auf die Einführeinrichtung 5 einwirkt, bildet die bewegliche Griffkomponente 12 ein Eingangs- bzw. Empfangsglied des Insertionskopfs. Wird das Aktivierungsglied 21 relativ zu dem Insertergehäuse 20 in die Vortriebsrichtung V bewegt, gleitet die Führungskurve 21a über das Eingriffselement 12a, d. h. über die das Eingriffselement 12a bildende Kontaktfläche des Empfangsglieds, d. h. der beweglichen Griffkomponente 12. Durch den Druckkontakt und den zur Vortriebsrichtung V geneigten Verlauf der Führungskurve 21a bewegt sich die Griffkomponente 12 quer zu der Vortriebsrichtung V auf die andere Griffkomponente 10 zu, und die Einführeinrichtung 5 schwenkt wie zum Insertionskopf als solchem beschrieben in die Einführposition. Die bewegliche Griffkomponente 12 bildet das Eingriffselement 12a an ihrem von der Basis 1, 2 abgewandten oberen Ende, im Ausführungsbeispiel mit ihrer äußeren Kante. Die Führungskurve 21a ist der Unterseite U21 des Inserters zugewandt. Die Neigung ist so gewählt, dass sich die Führungskurve 21a von einem von der Unterseite U21 abgewandten Ende in Vortriebsrichtung V von dem Insertionskopf bzw. der im aktivierten Zustand ausgeschwenkten Einführeinrichtung 5 oder der zentralen Längsachse des Inserters weg neigt. Der Neigungswinkel ist überall konstant, die Führungskurve 21a ist eine Schräge, d. h. eine schräge Linie oder Fläche.

Für die praktische Handhabung bietet es sich an, dass der Benutzer den Inserter nach dem Aufnehmen des Insertionskopfs mit der einen Hand am Aktivierungsglied 21 hält, beispielsweise indem er das Aktivierungsglied 21 umgreift, und mit der anderen Hand das Insertergehäuse 20 relativ zu dem gehaltenen Aktivierungsglied 21 gegen die Vortriebsrichtung V zieht. Auch dies wird als Betätigung des Aktivierungsglieds verstanden. Das Vortriebselement 22 und der Krafterzeuger 23 bewegen sich gemeinsam mit dem Insertergehäuse 20 relativ zu dem Aktivierungsglied 21. Der von der Halteeinrichtung in der Ausgangsposition gehaltene Insertionskopf wird mitgenommen, d. h. bewegt sich gleichermaßen relativ zu dem Aktivierungsglied 21 entgegen der Vortriebsrichtung V. Das Eingriffselement 12a gleitet längs der Führungskurve 21a. Über diese auf reinem Druckkontakt beruhende Schnittstelle wird die bewegliche Griffkomponente 12 quer zu der Vortriebsrichtung V bewegt, und die Einführeinrichtung 5 schwenkt in die Einführposition. Der Insertionskopf ist am Ende der Ausfahrbewegung, die das Insertionsgehäuse 20 und das Aktivierungsglied 21 relativ zueinander ausführen, aktiviert.

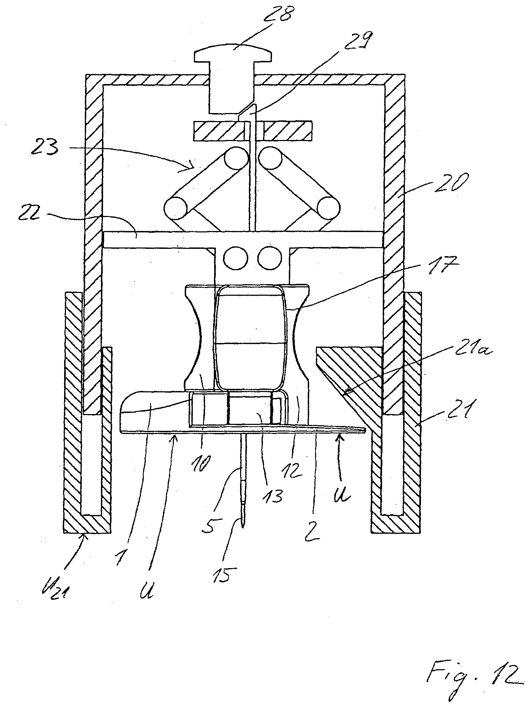

Figur 12 zeigt das System aus Inserter und Insertionskopf in dessen aktiviertem Zustand. Das Insertergehäuse 20 und das Aktivierungsglied 21 nehmen relativ zueinander die ausgefahrene Position ein. In dem ausgefahrenen Zustand umgeben die Wände des Insertergehäuses 20 und des Aktivierungsglieds 21 den aktivierten Insertionskopf bis über das freie Ende der Einführeinrichtung 5 und der Einstecheinrichtung 15 hinaus, d. h. die Spitze der Einstecheinrichtung 15 steht ein kleines Stück weit hinter der Unterseite U21 des Inserters zurück.

Das Insertergehäuse 20 und das Aktivierungsglied 21 sind in der ausgefahrenen Position relativ zueinander blockiert. Relativbewegungen in oder gegen die Vortriebsrichtung V sind in dem blockierten Zustand nicht möglich. Bei Erreichen der ausgefahrenen Position blockieren sich das Insertergehäuse 20 und das Aktivierungsglied 21 aneinander automatisch.

Für die Platzierung des Insertionskopfs setzt der Benutzer den Inserter auf der Hautoberfläche auf. Bei aufgesetztem Inserter drückt der Benutzer auf den Auslöser 28. Der Auslöser 28 wirkt über ein Kurvengelenk, im Ausführungsbeispiel ein einfaches Paar von Schrägen, auf das Blockierglied 29. Unter der Einwirkung des Auslösers 28 bewegt sich das Blockierglied 29 aus dem Blockiereingriff mit dem Insertergehäuse 20, so dass sich das Vortriebselement 22 unter der Einwirkung des Krafterzeugers 23 in die Vortriebsrichtung V bewegen kann. Der Krafterzeuger 23 beschleunigt das Vortriebselement 22 schlagartig. Das Vortriebselement 22 wirkt auf den Insertionskopf wie ein Hammer. Im ersten Abschnitt der Vortriebsbewegung federt die Haltefeder aus dem Halteeingriff mit der Haltestruktur 17 des Insertionskopfs, d. h. der Halteeingriff löst sich. Die Beschleunigung des Vortriebselements 22 in die Vortriebsrichtung V ist so groß, dass der reine Druckkontakt zwischen dem Vortriebselement 22 und dem Insertionskopf sicher erhalten bleibt, bis die Unterseite U des Insertionskopfs auf der gleichen Höhe wie die Unterseite U21 des Inserters und somit auf der Gewebeoberfläche platziert ist. Bereits zuvor durchdringt die Einstecheinrichtung 15 die Hautoberfläche, dringt in das Gewebe ein und nimmt dabei die Einführeinrichtung 5 mit.

Nachdem der Insertionskopf auf der Hautoberfläche platziert ist, greift der Benutzer den Griff 10, 12 und zieht ihn von der Basis 1, 2 ab. Dabei wird die Einstecheinrichtung 15 automatisch aus der Einführeinrichtung 5 heraus- und von der Basis 1, 2 abgezogen.

Um auch das Herausziehen der Einstecheinrichtung 15 zu automatisieren, bleibt der Halteeingriff zwischen der Halteeinrichtung des Inserters und der Haltestruktur 17 des Insertionskopfs in einer vorteilhaften Modifikation des Inserters bestehen und wird nicht, wie im beschriebenen Ausführungsbeispiel, durch die Beschleunigung des Vortriebselements 22 gelöst. In einer derartigen Modifizierung kann die Halteeinrichtung insbesondere ortsfest mit dem Vortriebselement 22 verbunden sein, so dass sie dessen Ausstoßbewegung in die Vortriebsrichtung V mitmacht. Um den Halteeingriff zu lösen, kann der Inserter mit einem Abstreifer ausgestattet sein, der nach dem Wegnehmen des Inserters vom Gewebe bei einem Zusammenschieben von Insertergehäuse 20 und Aktivierungsglied 21 automatisch den Insertionskopf aus dem Halteeingriff löst. Alternativ kann solch ein Abstreifer auch vollkommen unabhängig vom Aktivierungsglied 21 vorgesehen und separat betätigbar sein, um den Halteeingriff zu lösen.

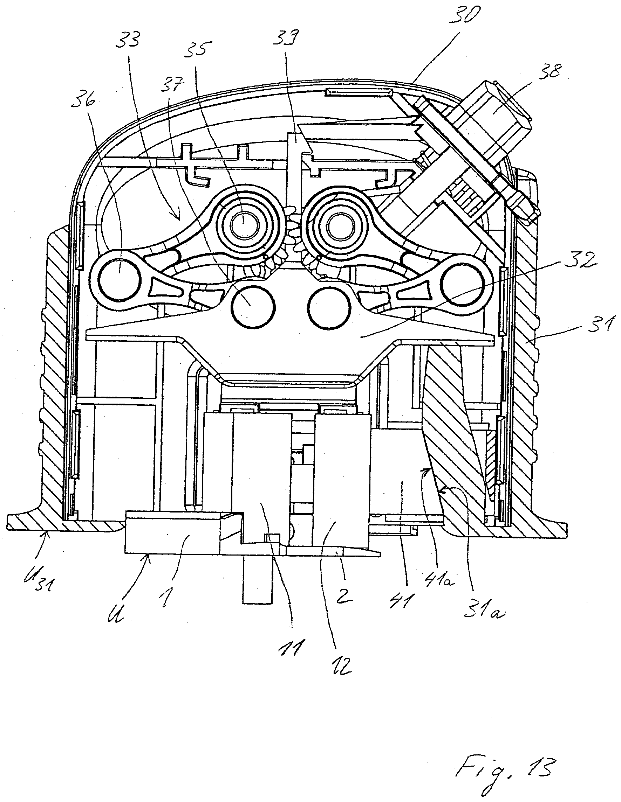

Die Figuren 13 bis 15 zeigen ein aus einem Insertionskopf und einem Inserter bestehendes System eines zweiten Ausführungsbeispiels. Der Insertionskopf ist derjenige der Figuren 7 bis 10, kann aber auch der gleiche wie im ersten Ausführungsbeispiel sein. Nur bei dem Inserter handelt es sich um eine Modifikation. Diejenigen Komponenten des Inserters des zweiten Ausführungsbeispiels, die in Bezug auf ihre Funktion mit den Komponenten des Inserters des ersten Ausführungsbeispiels vergleichbar sind, werden jeweils mit den um die Zahl zehn erhöhten Bezugszeichen des ersten Ausführungsbeispiels belegt. So gelten insbesondere zum Insertergehäuse 30 und dem Aktivierungsglied 31, was deren Form und Verbindung sowie Relativbeweglichkeit anbetrifft, die Ausführungen zum ersten Ausführungsbeispiel. Grundsätzlich das Gleiche gilt auch in Bezug auf das Vortriebselement 32, die Halteeinrichtung und der Krafterzeuger 33 sowie den Auslöser 38 und das Blockierglied 39. Soweit im Folgenden auf Unterschiede nicht hingewiesen wird und sich auch aus den Figuren nichts anderes ergibt, gelten die Ausführungen zum ersten Ausführungsbeispiel gleichermaßen auch für das zweite Ausführungsbeispiel.

Der Inserter des zweiten Ausführungsbeispiels unterscheidet sich von dem Inserter des ersten Ausführungsbeispiels im Wesentlichen im Hinblick auf das Gelenk, über welches das Aktivierungsglied 31 auf den Insertionskopf wirkt, um diesen durch die Aufziehbewegung des Insertergehäuses 30 relativ zu dem Aktivierungsglied 31 zu aktivieren. Im zweiten Ausführungsbeispiel bildet der Inserter selbst das Gelenk, nämlich mit zwei Gelenkelementen 31a und 41a, von denen eines das Aktivierungsglied 31 und das andere ein Effektorglied 41 bildet. Das Effektorglied 41 wird quer zu der Vortriebsrichtung V, im Ausführungsbeispiel rechtwinklig zu der Vortriebsrichtung V, hin und her beweglich von dem Insertionsgehäuse 30 gelagert. Das Gelenk 31a, 41a ist wieder ein Kurvengelenk. Die Führungskurve 31a entspricht der Führungskurve 21a des ersten Ausführungsbeispiels. Das Effektorglied 41 bildet das Eingriffselement 41a, das bei Verlängerung des Inserters an der Führungskurve 31a entlang gleitet und aufgrund des geneigten Verlaufs der Führungskurve 31a bei dem Aufziehen des Inserters eine Querbewegung des Effektorglieds 41 in Richtung auf die zentrale Längsachse des Inserters bewirkt. In dem Gelenk 31a, 41a wird somit die gegen die Vortriebsrichtung V weisende Bewegung, die das Insertergehäuse 30 bei dem Aufziehen relativ zu dem Aktivierungsglied 31 ausführt, in die Querbewegung des Effektorglieds 41 umgewandelt. Dessen Gelenkelement bzw. Eingriffselement 41a ist selbst in der Art einer Führungskurve gebildet, wird hier getriebetechnisch jedoch als Eingriffselement bezeichnet. Das Eingriffselement 41a könnte alternativ auch als beispielsweise einfacher Nocken oder Noppen geformt sein. Ebenso könnte das Eingriffselement 41a als Führungskurve bezeichnet und in einer anderen Modifikation das Gelenkelement 31a als vorragender Nocken oder Noppen geformt werden.

Die Schnittstelle, über welche der Inserter den Insertionskopf aktiviert, ist wieder als reiner Druckkontakt gebildet und besteht zwischen dem Effektorglied 41 und dem Empfangsglied bzw. der beweglichen Griffkomponente 12 des Insertionskopfs. Dieser reine, man könnte auch sagen lose Druckkontakt vereinfacht die Handhabung, da für die Aktivierung keine besondere Gelenkverbindung hergestellt werden muss, es genügt das Aufnehmen des Insertionskopfs in Kombination mit der Betätigung des Aktivierungsglieds 31, was in den Ausführungsbeispielen durch die Aufziehbewegung erfolgt. Der Druckkontakt, d. h. die von dem Effektorglied 41 ausgeübte Druckkraft, wirkt auf die bewegliche Griffkomponente 12 parallel zu der Richtung ihrer Beweglichkeit relativ zu der Basis 1, 2. Durch Zwischenschaltung des Effektorglieds 41 und Verlagerung des Gelenks 31a, 41a gänzlich zum Inserter wird auf die Griffkomponente 12 im zweiten Ausführungsbeispiel vorteilhafterweise quer zu der Richtung der Beweglichkeit der Griffkomponente 12 keine Kraft ausgeübt.

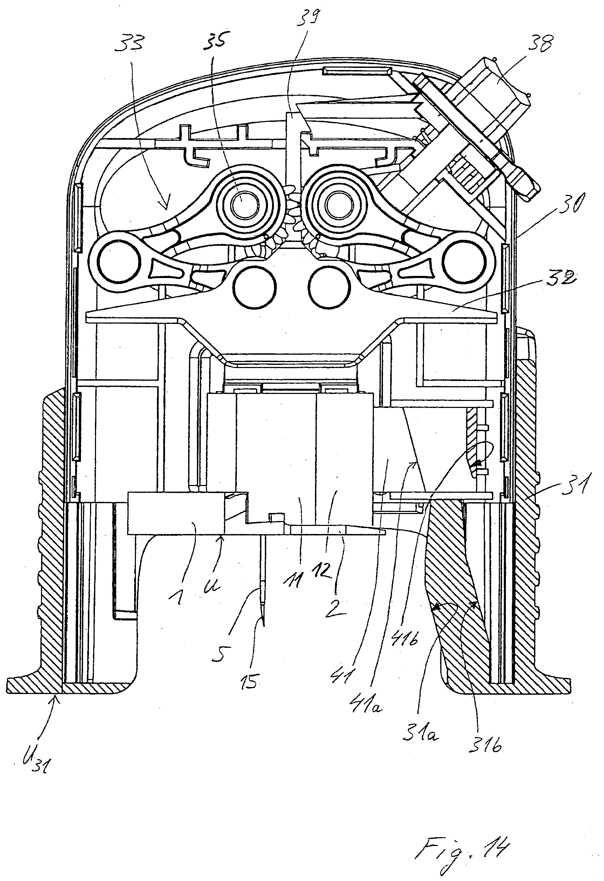

Figur 14 zeigt das System mit aktiviertem Insertionskopf. Im Verlaufe der Aufziehbewegung des Insertergehäuses 30, was auch als Betätigung des Aktivierungsglieds 31 verstanden wird, wurden die Einfuhreinrichtung 5 und die Einstecheinrichtung 15 in die Einführposition geschwenkt, so dass ihre gemeinsame Längsachse in die Vortriebsrichtung V weist. Die bewegliche Griffkomponente 12 hat wie zum Insertionskopf beschrieben die Verbindung zwischen dem Griff 10, 12 und der Basis 1, 2 gelöst. Der zwischen der Einführeinrichtung 5 und der Einstecheinrichtung 15 bestehende Reibschluss hält jedoch die Basis 1, 2 wie im ersten Ausführungsbeispiel an dem im Halteeingriff befindlichen Griff 10, 12.

Durch Betätigung des Auslösers 38 wird der Blockiereingriff, in dem sich das Blockierglied 39 noch mit dem Insertergehäuse 30 oder einer damit fest verbundenen Struktur befindet, gelöst und der Krafterzeuger 33 beschleunigt das Vortriebselement 32 in die Vortriebsrichtung V. Die Beschleunigung erfolgt wieder schlagartig, so dass auch die Antriebseinrichtung 32, 33 des zweiten Ausführungsbeispiels in der Art eines Hammers wirkt. Die Antriebskraft wird von zwei Schenkelfedern erzeugt, von denen je eine auf eines der beiden Schenkelpaare wirkt. Die im ortsfesten Drehlager 35 befestigten Schenkel 24 sind über einen Zahneingriff miteinander gekoppelt, der für eine synchrone Ausfahrbewegung der beiden Schenkelpaare sorgt.

Um den Inserter nach Platzierung des Insertionskopfs für eine erneute Verwendung bereitmachen zu können, muss das Effektorglied 41 aus der in Figur 14 gezeigten Endposition wieder zurück in die in Figur 13 gezeigte Endposition bewegt werden. Für diese Rückholbewegung bilden das Aktivierungsglied 31 und das Effektorglied 41 ein weiteres Gelenk 31b, 41b, das im Ausführungsbeispiel ebenfalls ein Kurvengelenk ist. Das Aktivierungsglied 31 bildet für das weitere Gelenk die Führungskurve 31b, und das Effektorglied 41 bildet das Eingriffselement 41b. Die Führungskurve 31b verläuft zumindest im Wesentlichen parallel zu der Führungskurve 31a. Die Führungskurven 31a und 31b sind an dem inneren Hülsenteil des Aktivierungsglieds 31 gebildet, die Führungskurve 31a an der Innenfläche und die Führungskurve 31b an der Außenfläche des inneren Hülsenteils. Sie liegen sich in etwa auf der gleichen Höhe, bezogen auf die Vortriebsrichtung V, gegenüber. Das Eingriffselement 41b liegt dem Eingriffselement 41a ebenfalls gegenüber mit einem lichten Abstand, so dass das innere Hülsenteil des Aktivierungsglieds 31 zwischen den beiden Eingriffselementen 41a und 41b ein- und ausfahren kann.

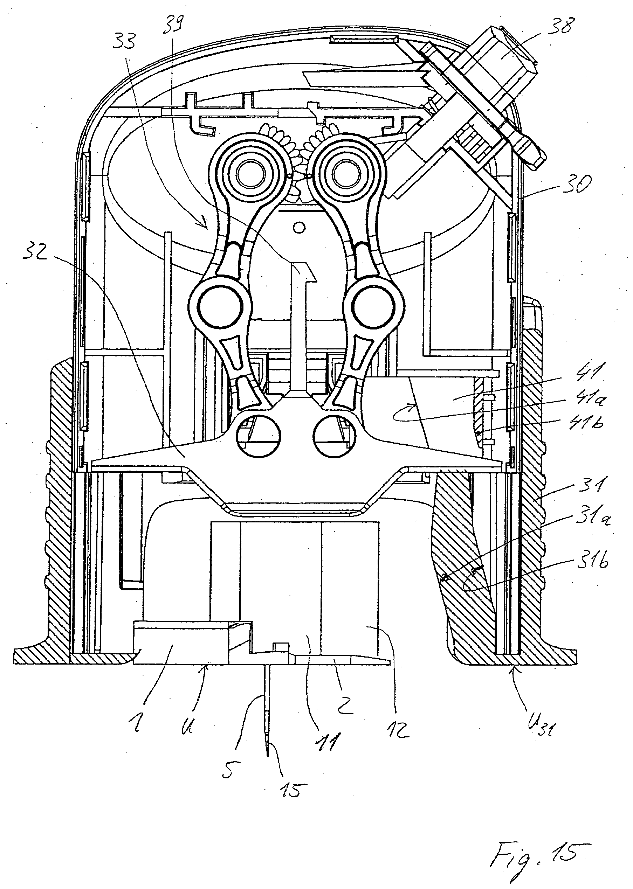

Figur 15 zeigt das System des zweiten Ausführungsbeispiels mit dem platzierten Insertionskopf. Der Inserter wird von dem Insertionskopf entfernt. Anschließend zieht der Benutzer den Griff 10, 12 von der Basis 1, 2 ab und schließt den Insertionskopf an einen Katheter einer Infusionspumpe an. In einer auch bereits zum ersten Ausführungsbeispiel erwähnten Modifikation, in welcher die Halteeinrichtung ortsfest mit dem Vortriebselement 32 verbunden ist und demgemäß den Griff 10, 12 noch halten kann, werden der Inserter und damit gemeinsam der noch gehaltene Griff 10, 12 von der Basis entfernt. Anschließend wird vorzugsweise mittels eines zusätzlichen Abstreifers der Halteeingriff gelöst und der Griff 10, 12 mit der Einstecheinrichtung 15 oder nur dieser alleine entsorgt.

Um den Inserter für die Verwendung mit einem weiteren Insertionskopf vorzubereiten, schiebt der Benutzer das Insertergehäuse 30 und das Aktivierungsglied 31 wieder zusammen in die eingefahrene Position, wie sie mit eingesetztem Insertionskopf in Figur 13 dargestellt ist. Bei der Einfahrbewegung fährt das innere Hülsenteil des Aktivierungsglieds 31 zwischen die Eingriffselemente 41a und 41b des Effektorglieds 41. Bei dieser Einfahrbewegung entsteht die weitere Gelenkverbindung zwischen der Führungskurve 31b und dem Eingriffselement 41b. Bei der Einfahrbewegung wird somit in dem Gelenk 31b, 41b das Effektorglied 41 wieder in die in Figur 13 eingenommene Endposition zurückbewegt, d. h. in Bezug auf die zentrale Längsachse des Inserters quer, vorzugsweise radial, nach auswärts bewegt.

Das unter der Einwirkung der Federeinrichtung 33 in die Vortriebsrichtung V ausgefahrene Vortriebselement 32 liegt einer Stirnseite des inneren Hülsenteils, die von der Unterseite U31 des Aktivierungsglieds 31 abgewandt ist, gegenüber. Durch Anschlagkontakt gegen diese Stirnseite wird die Vortriebsbewegung des Vortriebselements 32 gestoppt. Das Aktivierungsglied 31 ist geometrisch so bemessen, dass es in der ausgefahrenen Position des Teleskops 30, 31 das Vortriebselement 32 genau dann stoppt, wenn die Unterseite U des Insertionskopfs die Höhe der Unterseite U31 erreicht hat und daher bei aufgesetztem Inserter die Hautoberfläche gerade kontaktiert. Bei der Einfahrbewegung des Insertergehäuses 30 relativ zum Aktivierungsglied 31 bzw. des Aktivierungsglieds 31 relativ zu dem Insertergehäuse 30 wird das Vortriebselement 32 wegen des Anschlagkontakts vom Aktivierungsglied 31 gegen die Kraft des Krafterzeugers 33 tiefer in das Insertergehäuse 30 gedrückt, bis das Blockierglied 39 wieder im Blockiereingriff ist, wie ihn beispielhaft die Figuren 13 und 14 zeigen.

### Bezugszeichen:

- 1: Basis, Aufnahme
- 2: Basis, Flachteil
- 3: Ausnehmung
- 4: Ausnehmung
- 5: Einführeinrichtung
- 6: Gelenkelement
- 6a: Gelenkelementgegenstück
- 7: Zuführung
- 8: Zahnrad
- 9: Verbindungselement
- 10: Erste Griffkomponente
- 11: Erste Griffkomponente
- 12: Zweite Griffkomponente, Empfangsglied
- 12a: Gelenkelement, Eingriffselement, Druckkontaktfläche
- 13: Lasche, Pflaster
- 14: Aufnahme
- 15: Einstecheinrichtung
- 16: Verbindungselement
- 16a: Federsteg
- 16b: Verbindungseinrichtung
- 17: Haltestruktur
- 17a: Nadelhalter
- 17b: Kanülengehäuse
- 18: Zahnstange
- 19: Verbindungselement
- 20: Insertergehäuse
- 21: Aktivierungsglied
- 21a: Gelenkelement, Führungskurve
- 22: Vortriebselement
- 23: Krafterzeuger
- 24: Schenkel
- 25: Drehgelenk
- 26: Drehgelenk
- 27: Drehgelenk
- 28: Auslöser
- 29: Blockierglied
- 30: Insertergehäuse
- 31: Aktivierungsglied
- 31a: Gelenkelement, Führungskurve
- 31 b: Gelenkelement, Führungskurve
- 32: Vortriebselement
- 33: Krafterzeuger
- 34: Schenkel
- 35: Drehgelenk
- 36: Drehgelenk
- 37: Drehgelenk
- 38: Auslöser
- 39: Blockierglied
- 41: Effektorglied
- 41a: Gelenkelement, Eingriffselement
- 41b: Gelenkelement, Eingriffselement
- 42: Kulissenführung
- 42a: Schwenkführungsabschnitt
- 42b: Entrastungsführungsabschnitt
- 44: Kulissenstein, Zapfen
- 46: Sicherungsstruktur
- 48: Eingriffselement
- 49: Eingriffsausnehmung
- 50: Sicherungskulisse
- 50a: Sicherungsschulter
- 52: Steuerrampe
- 54: Auslenkrampe
- 56, 58: Septum
- 60: Rastschulter
- 62: Führung
- 64: Konnektor
- U: Unterseite
- V: Vortriebsrichtung

## Patentansprüche

1. Inserter zur Platzierung eines Insertionskopfes, wobei der Insertionskopf umfasst:
i) eine Basis (1, 2), mit einer auf organischem Gewebe platzierbaren Unterseite (U),
ii) eine in das Gewebe einführbare, von der Basis beweglich gelagerte Einführungseinrichtung (5),
iii) wobei die Einführungseinrichtung relativ zu der Basis aus einer Schutzposition, in der ein freies Ende der Einführeinrichtung hinter der Unterseite der Basis zurücksteht, in eine Einführungsposition beweglich ist, in der das freie Ende über die Unterseite vorragt,
iv) einen von der Basis abragenden Griff mit einer ersten Griffkomponente (10, 11) und einer relativ zu der Basis und der ersten Griffkomponente beweglichen zweiten Griffkomponente (12), und
v) eine Kopplung (8, 18, 42, 44), die eine Bewegung der zweiten Griffkomponente in eine Bewegung der Einführungseinrichtung überträgt,
der Inserter aufweisend:
a) ein Insertergehäuse (20, 30) aufnehmend eine Halteeinrichtung für einen zu platzierenden Insertionskopf, und einen Antrieb für den Insertionskopf, wobei der Antrieb ein Vortriebselement (22, 32) umfasst, das in und gegen eine Vortriebsrichtung (V) linear beweglich im Insertergehäuse angeordnet ist und einen Krafterzeuger (23, 33), der in der Vortriebsrichtung auf das Vortriebselement wirkt, wobei der Krafterzeuger zwei Paare von gelenkig miteinander verbundenen Schenkeln (24, 34) umfasst, die beiden Schenkelpaare symmetrisch zur Vortriebsrichtung angeordnet sind, jedes der beiden Schenkelpaare in einem zum Insertergehäuse ortsfesten Drehgelenk (25, 35) aufgehängt ist, die beiden Schenkel des jeweiligen Schenkelpaares in einem freien Drehgelenk (26, 36) miteinander drehbeweglich verbunden sind, und der von dem ortsfesten Gelenk abgewandte Schenkel jedes Schenkelpaares jeweils in einem weiteren Drehgelenk (27, 37) mit dem Vortriebselement verbunden ist, und
b) Mittel zur Bewegung der Einführungseinrichtung des Insertionskopfes in eine Einführungsposition,
**dadurch gekennzeichnet, dass**
die Mittel ein Aktivierungsglied (21, 31) sind, das eine Führungskurve (21a, 31a) bildet, welche zur Vortriebsrichtung (V) geneigt ist, wobei
- das Aktivierungsglied mit dem Insertergehäuse (20, 30) in und gegen die Vortriebsrichtung (V) beweglich verbunden ist,
- das Aktivierungsglied (21, 31) und der Insertionskopf ein Gelenk bilden, und
- die Gelenkelemente (41a, 41 b, 12a) des Gelenks durch die Führungskurve (21a, 31a) und durch ein von der beweglichen Griffkomponente (12) gebildetes Eingriffselement (12a, 41a) gebildet sind, wobei die Führungskurve (21a, 31a) über das Eingriffselement (12a, 41a) gleitet.

2. Inserter nach Anspruch 1, wobei das Aktivierungsglied (21) und ein von der Halteeinrichtung gehaltener Insertionskopf ein Gelenk bilden.

3. Inserter nach Anspruch 2, wobei das Gelenk ein Kurvengelenk mit zwei Gelenkelementen (12a, 21a, 31a, 41a) ist.

4. Inserter nach Anspruch 3, wobei die zwei Gelenkelemente die Führungskurve (21a) und ein von der beweglichen zweiten Griffkomponente (12) gebildetes Eingriffselement (1 2a) sind.

5. Inserter nach Anspruch 1, wobei der Inserter zusätzlich ein Effektorglied (41) umfasst, welches quer zur Vortriebsrichtung (V) hin und her beweglich von dem Insertergehäuse (30) gelagert ist.

6. Inserter nach Anspruch 5, wobei das Effektorglied (41) und das Aktivierungsglied (31) ein Gelenk mit zwei Gelenkelementen (31a, 41a) bilden.

7. Inserter nach Anspruch 6, wobei das Effektorglied (41) das Gelenkelement (41a) bildet.

8. Inserter nach Anspruch 7, wobei das Aktivierungsglied (31) eine Führungskurve (31a) bildet, welche zur Vortriebsrichtung (V) geneigt ist.

9. Inserter nach Anspruch 7, wobei das Gelenkelement (41a) als Nocken oder Noppen geformt ist.

10. Inserter nach Anspruch 7, wobei das Gelenkelement (31a) als vorragender Nocken oder Noppen ausgebildet ist.

11. Inserter nach einem der Ansprüche 5 bis 10, wobei das Effektorglied (41) und die bewegliche zweite Griffkomponente (12) eine Schnittstelle bilden, über welche der Inserter den Insertionskopf aktiviert.

12. System bestehend aus einem Insertionskopf umfassend:
i) eine Basis (1, 2), mit einer auf organischem Gewebe platzierbaren Unterseite,
ii) eine in das Gewebe einführbare, von der Basis beweglich gelagerte Einführungseinrichtung (5),
iii) wobei die Einführungseinrichtung relativ zu der Basis aus einer Schutzposition, in der ein freies Ende der Einführeinrichtung hinter der Unterseite (U) der Basis zurücksteht, in eine Einführungsposition beweglich ist, in der das freie Ende über die Unterseite vorragt,
iv) einen von der Basis abragenden Griff mit einer ersten Griffkomponente (10, 11) und einer relativ zu der Basis und der ersten Griffkomponente beweglichen zweiten Griffkomponente (12), und
v) eine Kopplung (8, 18, 42, 44), die eine Bewegung der zweiten Griffkomponente in eine Bewegung der Einführungseinrichtung überträgt,
und einem Inserter nach einem der vorangehenden Ansprüche.

## Claims

1. An inserter for placing an inserter head, the inserter head comprising:
i) a base (1, 2) comprising a lower side (U) which can be placed on organic tissue,
ii) an insertion means (5) which is movably mounted by the base and can be inserted into the tissue,
iii) wherein the insertion means can be moved relative to the base from a protective position, in which a free end of the insertion means is set back behind the lower side of the base, to an insertion position in which the free end protrudes beyond the lower side,
iv) a handle projecting from the base and having a first handle component (10, 11) and a second handle component (12) movable relative to the base and the first handle component; and
v) a coupling (8, 18, 42, 44) that transmits a movement of the second handle component into a movement of the insertion means,
the inserter comprising:
a) an inserter casing (20, 30) accommodating a holding means for an inserter head to be placed, and a drive for the insertion head, the drive comprising an advancing element (22, 32) arranged in the inserter casing linearly movable in and counter to an advancing direction (V), and a force generator (23, 33) acting on the advancing element in the advancing direction, wherein the force generator comprises two pairs of legs (24, 34) connected to one another via a joint, the two pairs of legs are arranged symmetrically with respect to the advancing direction, each of the two pairs of legs is suspended in a rotary joint (25, 35) which is stationary with respect to the inserter casing, the two legs of the respective pair of legs are rotatably connected to one another in a free rotary joint (26, 36), and that leg of each pair of legs which faces away from the stationary joint is connected to the advancing element in each case in a further rotary joint (27, 37), and
b) means for moving the insertion means of the insertion head into an insertion position,
**characterized in that**
the means are an activating member (21, 31) forming a guiding cam (21a, 31a) which is inclined with respect to the advancing direction (V), wherein
- the activating member is movably connected to the inserter casing (20, 30) in and counter to the advancing direction (V),
- the activating member (21, 31) and the insertion head form a joint, and
- the joint elements (41a, 41b, 12a) of the joint are formed by the guiding cam (21a, 31a) and by an engaging element (12a, 41a) formed by the movable handle component (12), wherein the guiding cam (21a, 31a) slides over the engaging element (12a, 41a) .

2. The inserter according to claim 1, wherein the activating member (21) and an insertion head held by the holding means form a joint.

3. The inserter according to claim 2, wherein the joint is a cam joint with two joint elements (12a, 21a, 31a, 41a) .

4. The inserter according to claim 3, wherein the two joint elements are the guiding cam (21a) and an engaging element (12a) formed by the movable second handle component (12).

5. The inserter according to claim 1, wherein the inserter additionally comprises an effector member (41) which is mounted by the inserter casing (30) to be movable back and forth transverse to the advancing direction (V).

6. The inserter according to claim 5, wherein the effector member (41) and the activating member (31) form a joint with two joint elements (31a, 41a).

7. The inserter according to claim 6, wherein the effector member (41) forms the joint element (41a).

8. The inserter according to claim 7, wherein the activating member (31) forms a guiding cam (31a) which is inclined with respect to the advancing direction (V) .

9. The inserter according to claim 7, wherein the joint element (41a) is shaped as a cam or burl.

10. The inserter according to claim 7, wherein the joint element (31a) is shaped as a protruding cam or burl.

11. The inserter according to any one of claims 5 to 10, wherein the effector member (41) and the movable second handle component (12) form an interface via which the inserter activates the insertion head.

12. A system consisting of an insertion head comprising:
i) a base (1, 2), comprising a lower side which can be placed on organic tissue,
ii) an insertion means (5) which is movably mounted by the base and can be inserted into the tissue,
iii) wherein the insertion means can be moved relative to the base from a protective position, in which a free end of the insertion means is set back behind the lower side (U) of the base, to an insertion position in which the free end protrudes beyond the lower side,
iv) a handle projecting from the base and having a first handle component (10, 11) and a second handle component (12) movable relative to the base and the first handle component; and
v) a coupling (8, 18, 42, 44) which transmits a movement of the second handle component into a movement of the insertion means,
and an inserter according to any one of the preceding claims.

## Revendications

1. Inséreuse, destinée à placer une tête d'insertion, la tête d'insertion comprenant :
i) une base (1, 2), dotée d'une face inférieure (U) susceptible d'être placée sur un tissu organique,
ii) un système d'introduction (5), susceptible d'être introduit dans le tissu, logé en étant mobile par la base,
iii) le système d'introduction étant mobile par rapport à la base à partir d'une position de protection, dans laquelle une extrémité libre du système d'introduction est en retrait derrière la face inférieure de la base, dans une position d'introduction, dans laquelle l'extrémité libre saillit par-dessus la face inférieure,
iv) une poignée saillant à partir de la base, dotée d'un premier composant de poignée (10, 11) et d'un deuxième composant de poignée (12) mobile par rapport à la base et au premier composant de poignée, et
v) un couplage (8, 18, 42, 44), qui transmet un déplacement du deuxième composant de poignée en un déplacement du système d'introduction,
l'inséreuse comportant :
a) un boîtier d'inséreuse (20, 30) recevant un système de maintien pour une tête d'insertion à placer, et un entraînement pour la tête d'insertion, l'entraînement comprenant un élément moteur (22, 32), qui est placé en étant linéairement mobile dans le boîtier d'inséreuse dans et à l'encontre d'une direction motrice (V) et un générateur de force (23, 33), qui agit dans la direction motrice sur l'élément moteur, le générateur de force comprenant deux paires de branches (24, 34) assemblées l'une à l'autre de manière articulée, les deux paires de branches étant placées de manière symétrique par rapport à la direction motrice, chacune des deux paires de branches étant accrochée dans une articulation rotative (25, 35) stationnaire par rapport au boîtier d'inséreuse, les deux branches de la paire de branches concernée étant assemblées l'une à l'autre en étant mobiles en rotation dans une articulation rotative (26, 36) libre, et la branche de chaque paire de branches qui est opposée à l'articulation stationnaire étant assemblée chaque fois dans une articulation rotative (27, 37) supplémentaire avec l'élément moteur, et
b) des moyens pour le déplacement du système d'introduction de la tête d'insertion dans une position d'introduction,
**caractérisée en ce que**
les moyens sont un organe d'activation (21, 31), qui forme une courbe de guidage (21a, 31a), laquelle est inclinée vers la direction motrice (V),
- l'organe d'activation étant assemblé avec le boîtier d'inséreuse (20, 30) en étant mobile dans et à l'encontre de la direction motrice (V),
- l'organe d'activation (21, 31) et la tête d'insertion formant une articulation, et
- les éléments articulés (41a, 41b, 12a) de l'articulation étant formés par la courbe de guidage (21a, 31a) et par un élément d'engagement (12a, 41a) formé par le composant de poignée (12) mobile, la courbe de guidage (21a, 31a) coulissant par-dessus l'élément d'engagement (12a, 41a).

2. Inséreuse selon la revendication 1, l'organe d'activation (21) et une tête d'insertion maintenue par le système de maintien formant une articulation.

3. Inséreuse selon la revendication 2, l'articulation étant une articulation à courbe, dotée de deux éléments articulés (12a, 21a, 31a, 41a).

4. Inséreuse selon la revendication 3, les deux éléments articulés étant la courbe de guidage (21a) et un élément d'engagement (12a) formé par le deuxième composant de poignée (12) mobile.

5. Inséreuse selon la revendication 1, l'inséreuse comprenant additionnellement un organe effecteur (41), lequel est logé en étant mobile en aller et retour à la transversale de la direction motrice (V) par le boîtier d'inséreuse (30).

6. Inséreuse selon la revendication 5, l'organe effecteur (41) et l'organe d'activation (31) formant une articulation dotée de deux éléments articulés (31a, 41a) .

7. Inséreuse selon la revendication 6, l'organe effecteur formant (41) l'élément articulé (41a).

8. Inséreuse selon la revendication 7, l'organe d'activation (31) formant une courbe de guidage (31a), laquelle est inclinée vers la direction motrice (V).

9. Inséreuse selon la revendication 7, l'élément articulé (41a) étant conçu sous la forme de came ou de téton.

10. Inséreuse selon la revendication 7, l'élément articulé (31a) étant conçu sous la forme de came ou de téton saillant(e) .

11. Inséreuse selon l'une quelconque des revendications 5 à 10, l'organe effecteur (41) et le deuxième composant de poignée (12) mobile formant une interface, par l'intermédiaire de laquelle l'inséreuse active la tête d'insertion.

12. Système, constitué d'une tête d'insertion comprenant :
i) une base (1, 2), dotée d'une face inférieure susceptible d'être placée sur un tissu organique,
ii) un système d'introduction (5), susceptible d'être introduit dans le tissu, logé en étant mobile par la base,
iii) le système d'introduction étant mobile par rapport à la base à partir d'une position de protection, dans laquelle une extrémité libre du système d'introduction est en retrait derrière la face inférieure (U) de la base, dans une position d'introduction, dans laquelle l'extrémité libre saillit par-dessus la face inférieure,
iv) une poignée saillant à partir de la base, dotée d'un premier composant de poignée (10, 11) et d'un deuxième composant de poignée (12) mobile par rapport à la base et au premier composant de poignée, et
v) un couplage (8, 18, 42, 44), qui transmet un déplacement du deuxième composant de poignée en un déplacement du système d'introduction,
et une inséreuse selon l'une quelconque des revendications précédentes.
